# EUROPÄISCHE PATENTSCHRIFT

(11) **EP 1 349 576 B1**
(45) Veröffentlichungstag und Bekanntmachung des Hinweises auf die Patenterteilung: **23.11.2011**
(21) Anmeldenummer: 01984881.1
(22) Anmeldetag: 21.12.2001
(51) Int. Cl.: A61P 37/06, A61P 17/00, A61K 38/55, A61K 45/06

(54) **KOMBINIERTE VERWENDUNG VON ENZYMINHIBITOREN ZUR THERAPIE UND PRÄVENTION ALLERGISCHER REAKTIONEN VOM TYP I NACH GELL UND COOMBS UND ZUR THERAPIE UND PRÄVENTION DERMATOLOGISCHER ERKRANKUNGEN MIT FOLLIKULÄREN UND EPIDERMALEN HYPERKERATOSEN UND EINER VERSTÄRKTEN KERATINOZYTENPROLIFERATION**
COMBINED USE OF ENZYME INHIBITORS FOR THE TREATMENT AND PREVENTION OF ALLERGIC REACTIONS OF TYPE I ACCORDING TO THE GELL AND COOMBS CLASSIFICATION, AND FOR THE TREATMENT AND PREVENTION OF DERMATOLOGICAL DISEASES ASSOCIATED WITH FOLLICULAR AND EPIDERMAL HYPERKERATOSIS AND REINFORCED KERATINOCYTE PROLIFERATION
UTILISATION COMBINÉE D'INHIBITEURS ENZYMATIQUES POUR LE TRAITEMENT ET LA PRÉVENTION DE REACTIONS ALLERGIQUES DE TYPE I SELON LA CLASSIFICATION DE GELL ET COOMBS ET POUR LE TRAITEMENT ET LA PRÉVENTION DE MALADIES DERMATOLOGIQUES ASSOCIÉES AVEC L'HYPERKÉRATOSE FOLLICULAIRE ET ÉPIDERMALE ET LA PROLIFERATION RENFORCÉE DES KÉRATINOCYTES

(30) Priorität: 02.01.2001 DE 10100052; 19.01.2001 DE 10102392; 09.11.2001 DE 10155093
(43) Veröffentlichungstag der Anmeldung: 08.10.2003
(73) Patentinhaber: IMTM GmbH, 39120 Magdeburg (DE)
(72) Erfinder: ANSORGE, Siegfried, 39291 Hohenwarte (DE); LENDECKEL, Uwe, c/o Universitätklinik Magdeburg, 39120 Magdeburg (DE); NEUBERT, Klaus, c/o M.Luther-Univ. Biochem./techn., 06120 Halle (DE); REINHOLD, Dirk, c/o Universitätsklinik Magdeburg, 39120 Magdeburg (DE); VETTER, Robert,c/o Universitätsklinik Magdeburg, 39120 Magdeburg (DE); GOLLNICK, Harald, c/o Universitätsklinik Magdeburg, 39120 Magdeburg (DE)
(74) Vertreter: Koepe, Gerd L.
(86) Internationale Anmeldenummer: PCT/EP2001/015199
(87) Internationale Veröffentlichungsnummer: WO 2002/053170

(56) Entgegenhaltungen:
- WO-A-01/89569
- DE-A- 19 826 972
- AUGUSTIJNS PATRICK F ET AL: "Transport and metabolism of delta sleep-inducing peptide in cultured human intestinal epithelial cell monolayers." DRUG METABOLISM AND DISPOSITION, Bd. 23, Nr. 12, 1995, Seiten 1372-1378, XP001094376 ISSN: 0090-9556
- STEINMETZER T ET AL: "PEPTIDYL AMMONIUM METHYL KETONES AS SUBSTRATE ANALOG INHIBITORS OF PROLINE-SPECIFIC PEPTIDASES" JOURNAL OF ENZYME INHIBITION, NEW YORK, NY, US, Bd. 7, Nr. 2, 1993, Seiten 77-85, XP001018795
- SHIMAZAWA R ET AL: "NOVEL SMALL MOLECULE NONPEPTIDE AMINOPEPTIDASE N INHIBITORS WITH A CYCLIC IMIDE SKELETON" JOURNAL OF ENZYME INHIBITION, NEW YORK, NY, US, Bd. 14, Nr. 4, 1999, Seiten 259-275, XP001018772
- REINHOLD D ET AL: "INHIBITORS OF DIPEPTIDYL PEPTIDASE IV INDUCE SECRETION OF TRANSFORMING GROWTH FACTOR-BETA1 IN PWM-STIMULATED PBMC AND T CELLS" IMMUNOLOGY, BLACKWELL SCIENTIFIC PUBLICATIONS, GB, Bd. 91, Nr. 3, 1997, Seiten 354-360, XP000867395 ISSN: 0019-2805 in der Anmeldung erwähnt
- ANSORGE S ET AL: "MEMBRANE-BOUND PEPTIDASES OF LYMPHOCYTES: FUNCTIONAL IMPLICATIONS" BIOMEDICA BIOCHIMICA ACTA, AKADEMIE VERLAG, BERLIN, DE, Bd. 50, Nr. 4 - 6, 1991, Seiten 799-807, XP001021975 ISSN: 0232-766X
- HOFFMANN T ET AL: "DIPEPTIDYL PEPTIDASE IV (CD 26) AND AMINOPEPTIDASE N (CD 13) CATALYZED HYDROLYSIS OF CYTOKINES AND PEPTIDES WITH N-TERMINAL CYTOKINE SEQUENCES" FEBS LETTERS, ELSEVIER SCIENCE PUBLISHERS, AMSTERDAM, NL, Bd. 336, Nr. 1, Dezember 1993 (1993-12), Seiten 61-64, XP000867397 ISSN: 0014-5793
- AUGUSTYNS K ET AL: "THE UNIQUE PROPERTIES OF DIPEPTIDYL-PEPTIDASE IV (DPP IV/CD26) AND THE THERAPEUTIC POTENTIAL OF DPP IV INHIBITORS" CURRENT MEDICINAL CHEMISTRY, BENTHAM SCIENCE PUBLISHERS BV, BE, Bd. 6, Nr. 4, 1999, Seiten 311-327, XP000870290 ISSN: 0929-8673

## Beschreibung

Die Erfindung betrifft die Hemmung der DNS-Synthese und damit der Proliferation von Immunzellen durch die kombinierte Wirkung von Inhibitoren der Aminopeptidase N (APN, EC3.4.11.2, CD13) und der Dipeptidylpeptidase IV bzw. durch die kombinierte Hemmung der Aktivität der genannten Enzyme im Ergebnis der simultanen Applikation von jeweils spezifischen Inhibitoren dieser Enzyme auf der Basis von Aminosäurederivaten, Peptiden oder Peptidderivaten, durch welche die Aktivierung, die DNS-Synthese und damit die Proliferation von Immunzellen supprimiert wird.

Die Erfindung betrifft auch die Hemmung der für die Proliferation notwendigen DNS-Synthese und der Zytokinproduktion (Interleukin-4, IL-4) von T_{H2}-Zellen durch die kombinierte Wirkung von Inhibitoren der Aminopeptidase N (APN, EC3.4.11.2, CD13), der Dipeptidylpeptidase IV (DP IV, EC 3.4.14.5, CD26) im Ergebnis der simultanen Applikation von jeweils spezifischen Inhibitoren dieser Enzyme auf der Basis von Aminosäurederivaten, Peptiden oder Peptidderivaten, durch welche die Aktivierung, die Proliferation (DNS-Synthese) und Zytokinproduktion (IL-4) von T_{H2}-Zellen supprimiert wird.

Die Erfindung betrifft auch die Hemmung der für die Proliferation notwendigen DNS-Synthese von Keratinozyten durch die kombinierte Wirkung von Inhibitoren der Aminopeptidase N (APN, EC3.4.11.2, CD 13) und der Dipeptidylpeptidase IV (DP IV, EC 3.4.14.5, CD26) im Ergebnis der simultanen und zeitlich unmittelbar aufeinanderfolgenden Applikation von jeweils spezifischen Inhibitoren dieser Enzyme oder ähnlich wirkender Enzyme auf der Basis von Aminosäurederivaten, Peptiden oder Peptidderivaten, durch welche die Proliferation (DNS-Synthese) von Keratinozyten supprimiert wird.

Augustijns Patrick F. et al. in Drug Metabolism and Disposition 23, Nr. 12 (1995), 1372 - 1378] offenbart, dass die Kombination von Diprotin A (DP IV-Inhibitor) und Bestatin (APN-Inhibitor) zu einer Stabilitätserhöhung des Enzyms DSIP (Delta-Sleep-inducing peptide) führt.

Steinmetzer Torsten et al. in Journal of Enzyme Inhibition, New York 7, Nr. 2 (1993), 77 - 85 offenbart die Inkubation von PEP und DP IV mit Peptidylammoniummethyl- und Pyridiniummethyl-Ketonen in Gegenwart von Substrat, was zur Bildung von nicht-kovalenten Inhibitor-Komplexen führt.

Shimazawa R. et al. in J. Enzyme Inhibition 14 (1999), 259 - 275 beschreibt die Eigenschaft von Molekülen mit einem cyclischen Imid-Grundgerüst als Aminopeptidase-Inhibitoren.

Reinhold D. et al. in Immunology, Blackwell Scientific Publications 91, Nr. 3 (1997) 354 - 360 offenbart, dass die Inkubation mit zwei DP IV-Inhibitoren, nämlich Lys(Z(NO2))-thiazolidid und Lys(Z(NO2))-piperidid zur Hemmung der DNS-Synthese von PWMstimulierten gereinigten T-Zellen führt.

Ansorge S. et al. in Biomed. Biochim Acta 50 (191), 4 - 6 beschreibt die Rolle zweier Enzyme, nämlich der beiden Peptidasen DP IV und APN, im Organismus sowie ihre getrennte Inhibierung.

Hoffmann T. et al in FEBS Letters, Elsevier Science Publishers, 336, Nr. 1 (1993) 61 - 64 offenbart den Abbau/die Hydrolyse von Cytokin-analogen Oligopeptiden mittels Dipeptidylpeptidase IV und Aminopeptidase N.

Die WO 01/89569 beschreibt, dass zur Therapie von Autoimmun- und chronischen Erkrankungen mit entzündlicher Genese sowie zur Behandlung von Abstoßungsepisoden nach Transplantationen die gleichzeitige Applikation von Hemmstoffen der Enzymaktivitäten von Alanyl-Aminopeptidase und Dipeptidylpeptidase IV bzw. entsprechender Zubereitungen und Darreichungsformen daraus geeignet sind..

Die DE 19826972 offenbart die Verwendung von DP IV-Inhibitoren zur Therappie von entzündlichen und nicht-entzündlichen epidermalen Hyperproliferationszuständen, benignen und malignen umschriebenen epidermalen clonalen Expansionen, benignen und malignen Hyperproliferationszuständen, Nagelzeilhyperproliferationen etc.

Die WO 00/10549 betrifft die Verwendung von DP IV-Inhibitoren bei der Behandlung von Entzündungen, Arteriolosklerose, Allergien etc.

Meske S. et al. in Zeitschrift für Rheumatologie (1985), Band 44, Nr. 5, Seiten 231 - 236 offenbart die Verwendung des Immunmodulans Bestatin bei chronischer Polyarthritis.

Thestrup-Pedersen K. et al. in Acta Dermato-Venereologica (1983), Band 63, Nr. 6, Seiten 549 bis 552 beschreibt die Verwendung von Bestatin bei Patienten mit atopischer Dermatitis.

Aus der Derwent-Zusammenfassung von JP 10218789 geht hervor, daß Ubenimex (Bestatin) immunologische Antworten verstärkt und allergische Reaktionen hemmt.

Für alle Erkrankungen mit Autoimmunpathogenese gilt, dass eine Aktivierung und Proliferation von Immunzellen, insbesondere von autoreaktiven T-Zellen, dem Krankheitsprozess zugrunde liegen bzw. diesen ausmachen. Ähnliche Mechanismen kommen bei einer Reihe von entzündlichen Erkrankungen wie der Atherosklerose zur Wirkung, wo T-Lymphozyten eine zentrale Rolle bei Enstehung und Chronifizierung des Krankheitsprozesses spielen.

Es ist gezeigt worden, dass im Prozess der Aktivierung und klonalen Expansion von Immunzellen, insbesondere von T-Lymphozyten, membranständige Peptidasen wie DP IV oder APN eine Schlüsselrolle spielen [Fleischer B: CD26 a surface protease involved in T-cell activation. Immunology Today 1994; 15:180-184; Lendeckel U et al.: Role of alanyl aminopeptidase in growth and function of human T cells. International Journal of Molecular Medicine 1999; 4:17-27; Riemann D et al.: CD13 - not just a marker in leukemia typing. Immunology Today 1999; 20:83-88]. Verschiedene Funktionen mitogen-stimulierter mononukleärer Zellen (MNZ) oder angereicherter T-Lymphozyten wie DNS-Synthese, Produktion und Sekretion von immunstimulierenden Zytokinen (IL-2, IL-6, IL-12, IFN-γ) und Helferfunktionen für B-Zellen (IgG- und IgM-Synthese) können in Gegenwart von spezifischen Inhibitoren der DP IV und der APN gehemmt werden [Schön E et al.: The dipeptidyl peptidase IV, a membrane enzyme involved in the proliferation of T lymphocytes. Biomed. Biochim. Acta 1985; 2: K9-K15; Schön E et al.: The role of dipeptidyl peptidase IV in human T lymphocyte activation. Inhibitors and antibodies against dipeptidyl peptidase IV suppress lymphocyte proliferation and immunoglobulin synthesis in vitro. Eur. J. Immunol. 1987; 17: 1821-1826; Reinhold D et al.: Inhibitors of dipeptidyl peptidase IV induce secretion of transforming growth factor β1 in PWM-stimulated PBMNC and T cells. Immunology 1997; 91: 354-360; Lendeckel U et al. : Induction of the membrane alanyl aminopeptidase gene and surface expression in human T-cells by mitogenic activation. Biochem. J. 1996; 319: 817-823; Kähne T et al.: Dipeptidyl peptidase IV: A cell surface peptidase involved in regulating T cell growth (Review). Int. J. Mol. Med. 1999; 4: 3-15; Lendeckel U et al.: Role of alanyl aminopeptidase in growth and function of human T cells (Review). Int. J. Mol. Med. 1999; 4: 17-27].

Auf der anderen Seite haben wissenschaftliche Erkenntnisse der letzten Jahre die Atherosklerose als eine entzündliche Erkrankung charakterisiert, wobei den T-Lymphozyten eine entscheidende Rolle für die Enstehung und Entwicklung der Erkrankung zukommt [Ross R: Atherosclerosis- an inflammatory disease. New Engl. J. Med. 1999; 340 (2):115-126]. Demnach werden atheriosklerotische Läsionen als eine Serie spezifischer zellulärer und molekularer Reaktionen verstanden, die zusammengenommen eindeutig als Entzündung zu charakterisieren sind. Solche Läsionen, die hauptsächlich in großen und mittleren elastischen und muskullösen Arterien vorkommen, führen zu Ischämie (Durchblutungsstörungen) von Herz, Him und Extremitäten bis hin zu Infarkten in den genannten Organen. Atherosklerotische Läsionen bilden sich an definierten arteriellen Orten, wo Abzweigungen und Kurven charakteristische Veränderungen des Blutflusses und der Scherkräfte sowie die Ausbildung von Turbulenzen bewirken [Gotlieb AI et al.: The role of rheology in atherosclerotic coronary artery disease. In: Fuster V, Ross R, Topol EJ, eds. Atherosclerosis and coronary athery disease. Vol. 1 Philadelphia: Lippincott-Raven, 1996: 595-606]. Gefäßendothel-Zellen bilden dann an diesen Orten spezifische Moleküle, die für die Attraktion, Bindung, Akkumulation sowie Aktivierung von T-Lymphozyten und Monozyten verantwortlich sind. T-Lymphozyten sind wesentliche inflammatorische Zellen in allen Phasen der Atherogenese. T-Zellen infiltrieren aus dem peripheren Blut in die atheriosklerotischen Plaques und vermehren sich am Ort der Läsion [Jonasson L et al.: Regional accumulation of T cells, macrophages and smooth muscle cells in the human atherosclerotic plaque. Arteriosclerosis. 1986; 6: 131-138; van der Wal AC et al.: Atherosclerotic lesions in humans: in situ immunophenotypic analysis suggesting an immune mediated response. Lab. Invest. 1989; 61: 166-170]. Im Ergebnis dieser Anhäufung aktivierter T-Lymphozyten, die sich durch eine starke Expression der Alanylaminopeptidase und der Dipeptidyl-Peptidase IV auszeichnen, am Ort der atherosklerotischen Läsion werden Chemokine, Zytokine, Wachstumsfaktoren und Proteasen freigesetzt, die zur weiteren Verstärkung des Krankheitsgeschehens führen, indem andere Immunzellen rekrutiert und aktiviert werden [Libby P and Ross R. Cytokines and growth regulatory molecules. In: Fuster V, Ross R, Topol EJ, eds. Atherosclerosis and coronary athery disease. Vol. 1 Philadelphia: Lippincott-Raven, 1996: 585-594].

Auch Monozyten, die in atherosklerotischen Plaques lokalisiert sind, zeichnen sich durch die konstitutive Expression von z. B. Alanylaminopeptidase (APN) aus und sind, wie unsere Arbeiten zeigen, nachhaltig durch Hemmstoffe der oben beschriebenen Enzyme in ihrem Wachstum und ihrer Funktion zu supprimieren. Gleiches gilt für Endothelzellen, die ebenfalls diese Ektopeptidasen exprimieren.

Dem Angiotensin-konvertierenden Enzym kommt eine besondere Rolle in der Pathogenese der Atherosklerose zu: Dieses Enzym bewirkt die Bildung des stark blutdrucksteigernden Angiotensin II (Ang II) aus dem Ang I. Hypertonie ist ein wichtiger Risikofaktor für Atherosklerose und betroffene Patienten haben oft erhöhte Ang II-Spiegel. Daneben ist Ang II proatherogen, indem es das Wachstum von glatten Muskeln (Gefäße) stimuliert [Chobanian AV et al. Renin angiotensin system and atherosclerotic vascular disease. In: Fuster V, Ross R, Topol EJ, eds. Atherosclerosis and coronary athery disease. Vol. 1 Philadelphia: Lippincott-Raven, 1996: 237-242; Gibbons GH et al. Vascular smooth muscle cell hypertrophy vs. hyperplasia: autocrine TGF-β1 expression determines growth response to angiotensin II. J Clin. Invest. 1992; 90: 456-461]. Ang II verstärkt die Entzündungsreaktion darüberhinaus auch über die Erhöhung der Lipoxygenase-Aktivität, wodurch entzündungsfördernde Mediatoren verstärkt freigesetzt werden.

Die vorliegende Erfindung betrifft die Verwendung von wenigstens einem Inhibitor, ausgewählt aus der Gruppe, die besteht aus Inhibitoren der Dipeptidylpeptidase IV (DP IV) und Inhibitoren von Enzymen mit gleicher Substratspezifität (DP IV-analoger Enzymaktivität), ausgewählt aus der Gruppe, die besteht aus
Xaa-Pro-Dipeptiden (Xaa = α-Aminosäure bzw. seitenkettengeschütztes Derivat), vorzugsweise Dipeptidphosphonsäurediarylester, Dipeptidboronsäuren (z.B. Pro-boro-Pro) oder deren Salzen, Xaa-Xaa-(Trp)-Pro-(Xaa)n-Peptiden (Xaa = α-Aminosäure, n = 0 - 10), oder deren Salzen bzw. Aminosäure (Xaa)-amiden oder deren Salzen, wobei Xaa eine α-Aminosäure bzw. ein seitenkettengeschütztes Derivat, vorzugsweise N^{ε}-4-Nitrobenzyloxycarbonyl-L-Lysin, L-Prolin, L-Tryptophan, L-Isoleucin, L-Valin ist und als Amidstruktur cyclische Amine, z.B. Pyrrolidin, Piperidin, Thiazolidin und deren Derivate fungieren,
in Kombination mit wenigstens einem Inhibitor, ausgewählt aus der Gruppe, die besteht aus Inhibitoren der Alanyl-Aminopeptidase (Aminopeptidase N, APN) und Inhibitoren von Enzymen mit gleicher Substratspezifität (APN-analoger Enzymaktivität),
ausgewählt aus der Gruppe, die besteht aus Actinonin, Leuhistin, Phebestin, Amastatin, Bestatin, Probestin, RB3014, β-Aminothiolen, α-Aminophosphinsäuren, vorzugsweise D-Phe-Ψ[PO(OH)-CH₂]-Phe-Phe, und deren Salzen, zur Herstellung eines Arzneimittels zur Vorbeugung und Therapie von allergischen Reaktionen vom Typ I.

Darüber hinaus betrifft die vorliegende Erfindung die Verwendung von wenigstens einem Inhibitor, ausgewählt aus der Gruppe, die besteht aus Inhibitoren der Dipeptidylpeptidase IV (DP IV) und Inhibitoren von Enzymen mit gleicher Substratspezifität (DP IV-analoger Enzymaktivität), ausgewählt aus der Gruppe, die besteht aus
Xaa-Pro-Dipeptiden (Xaa = α-Aminosäure bzw. seitenkettengeschütztes Derivat), vorzugsweise Dipeptidphosphonsäurediarylester, Dipeptidboronsäuren (z.B. Pro-boro-Pro) oder deren Salzen, Xaa-Xaa-(Trp)-Pro-(Xaa)n-Peptiden (Xaa = α-Aminosäure, n = 0 - 10), oder deren Salze bzw. Aminosäure (Xaa)-amiden oder deren Salze, wobei Xaa eine α-Aminosäure bzw. ein seitenkettengeschütztes Derivat, vorzugsweise N^{ε}-4-Nitrobenzyloxycarbonyl-L-Lysin, L-Prolin, L-Tryptophan, L-Isoleucin, L-Valin ist und als Amidstruktur cyclische Amine, z.B. Pyrrolidin, Piperidin, Thiazolidin und deren Derivate fungieren,
in Kombination mit wenigstens einem Inhibitor, ausgewählt aus der Gruppe, die besteht aus Inhibitoren der Alanyl-Aminopeptidase (Aminopeptidase N, APN) und Inhibitoren von Enzymen mit gleicher Substratspezifität (APN-analoger Enzymaktivität),
ausgewählt aus der Gruppe, die besteht aus Actinonin, Leuhistin, Phebestin, Amastatin, Bestatin, Probestin, RB3014, β-Aminothiolen, α-Aminophosphinsäuren, vorzugsweise D-Phe-Ψ[PO(OH)-CH₂]-Phe-Phe, und deren Salzen,
zur Herstellung eines Arzneimittels zur Vorbeugung und Therapie von dermatologischen Erkrankungen, die mit follikulären und epidermalen Hyperkeratosen und einer verstärkten Keratinozytenproliferation einhergehen, ausgewählt aus der Gruppe, die besteht aus entzündlichen epidermalen Hyperproliferationszuständen, ausgewählt aus der Gruppe, die besteht aus Lichen ruber und Subtypen, Parapsoriasis-Gruppe, Keratosis lichenoides, Lichen simplex chronicus und reaktive lichenoide, lichenoide Reaktionen bei GvHD, ILVEN-Naevus, Lupus erythematodes chron. disc./SCLE/SLE, Pityriasis rubra pilaris, M. Grover, Vitiligo und mit Hyperproliferation von Keratinozyten einhergehende Erythrodermien;
nicht entzündlichen epidermalen Hyperproliferationszuständen;
benignen und malignen umschriebenen epidermalen clonalen Expansionen (z. B. Warzen, Condylome, aktinische Keratosen/Präcancerosen);
benignen und malignen follikulären Hyperproliferationszuständen, ausgewählt aus der Gruppe, die besteht aus Keratosis follikularis, Ulerythema ophryogenes, Hypertrichosen, Trichilemmalcysten, Haarfollikelzelltumoren, proliferierenden Trichilemmalcysten und Mischtumoren;
benignen und malignen epithelialen Adnextumoren; und
primären und reaktiven Nagelzellhyperproliferationen.

Gemäß einer bevorzugten Verwendung werden als Inhibitoren der DP IV Aminosäureamide, z.B. N^{ε}-4-Nitrobenzyloxycarbonyl-L-Lysin-thiazolidid, -pyrrolidid und -piperidid sowie das entsprechende 2-Cyanothiazolidid-, 2-Cyanopyrrolidid- und 2-Cyanopiperidid-Derivat eingesetzt.

Entsprechend der erfindungsgemäßen Verwendung werden die Inhibitorkombinationen gemäß der vorstehenden Angaben eingesetzt zur Vorbeugung und Therapie von allergischen Reaktionen vom Typ I. Bevorzugt finden sie Anwendung bei der Vorbeugung und Therapie von Asthma bronchiale oder Heuschnupfen.

Die Inhibitorkombinationen werden simultan mit an sich bekannten Träger-, Hilfs- und/oder Zusatzstoffen verabreicht. Die Verabreichung erfolgt einerseits als systemische Anwendung zur oralen, transdermalen, intravenösen, subcutanen, intracutanen, intramuskulären, rektalen, vaginalen, sublingualen Applikation und andererseits als topische Anwendung in Form von z.B. Cremes, Salben, Pasten, Gelen, Lösungen, Sprays, Liposomen, Schüttelmixturen, Hydrokolloidverbänden, Pflaster und ähnlichen Trägersubstraten, Jet-Injektionen bzw. anderen dermatologischen Grundlagen/Vehikeln, einschließlich instillativer Applikation.

Alternativ dazu werden die Inhibitorkombinationen in der Art verwendet, bei der die Inhibitoren der Dipeptidylpeptidase IV (DP IV) sowie von Enzymen mit gleicher Substratspezifität (DP IV-analoge Enzymaktivität) und die Inhibitoren der Alanyl-Aminopeptidase (Aminopeptidase N, APN) sowie von Enzymen mit gleicher Substratspezifität (APNanaloge Enzymaktivität) in räumlich getrennter Formulierung in Kombination mit an sich bekannten Träger-, Hilfs- und/oder Zusatzstoffen gleichzeitig oder zeitlich unmittelbar aufeinanderfolgend mit dem Ziel einer gemeinsamen Wirkung verabreicht werden.

Der Erfindung liegt der überraschende Befund zugrunde, dass die gleichzeitige Wirkung von Inhibitoren der enzymatischen Aktivitäten bzw. die gleichzeitige Beeinflussung der biologischen Aktivitäten von Dipeptidylpeptidase IV sowie von Enzymen mit gleicher Sübstratspezifität (DP IV-analoger Enzymaktivität) und Aminopeptidase N sowie von Enzymen mit gleicher Substratspezifität (APN-analoger Enzymaktivität) die DNS-Synthese und damit die Proliferation von mononukleären Zellen (MNZ) als auch von T-Zellen in einem Ausmaß hemmt, das durch die einzelne Applikation dieser Enzyminhibitoren - auch bei höherer Dosierung - nicht erreicht werden kann. Obgleich die genannten Inhibitoren letztendlich den gleichen Prozess, nämlich die DNS-Synthese und damit die Proliferation von Immunzellen, beeinflussen, ist dieser Effekt bei einzelner Applikation der Inhibitoren wesentlich schwächer ausgeprägt und nicht dauerhaft. Wegen der funktionellen Überschneidung der enzymatischen Aktivitäten der genannten Enzyme resultiert, wie unsere Daten zeigen, eine additive bis superadditive Hemmwirkung auf DNS-Synthese und Proliferation aus der gleichzeitigen Hemmung von zwei oder mehreren dieser Enzyme.

Die Erfindung zeigt, dass zur Therapie von Autoimmunerkrankungen und entzündlichen Erkrankungen wie der Atherosklerose, für deren Enstehung die Proliferation und die Aktivierung von T-Lymphozyten eine zentrale Rolle Bedeutung hat, die gleichzeitige Applikation von Hemmstoffen der oben genannten Enzyme bzw. entsprechender Zubereitungen und Darreichungsformen daraus geeignet sind.

Im einzelnen liegen der Erfindung die Befunde zugrunde, dass die DNS-Synthese von MNZ und T-Zellen durch die simultane Administration von Inhibitoren der enzymatischen Aktivität von Dipeptidylpeptidase IV sowie von Enzymen mit gleicher Sustratspezifität (DP IV-analoger Enzymaktivität) und Aminopeptidase N sowie von Enzymen mit gleicher Substratspezifität (APN-analoger Enzymaktivität) in additiver bis superadditiver Weise inhibiert wird.

Die Applikation von Enzyminhibitoren stellt bei den genannten Erkrankungen eine neuartige Methode und ergänzende Therapieform dar.

Die erfindungsgemäß applizierten Inhibitoren der Dipeptidylpeptidase IV und der Aminopeptidase N, können in pharmazeutisch anwendbaren Formulierungskomplexen als Inhibitoren, Substrate, Pseudosubstrate, inhibitorisch wirkende Peptide und Peptidderivate dieses Enzyms zur Anwendung kommen. Bevorzugte Effektoren sind beispielsweise für die DP IV Xaa-Pro-Dipeptide, worin Xaa eine α-Aminosäure bzw. ein seitenkettengeschütztes Derivat ist, entsprechende Derivate, vorzugsweise Dipeptidphosphonsäurediarylester, Dipeptidboronsäuren (z.B. Pro-boro-Pro) und deren Salze, Xaa-Xaa-(Trp)-Pro-(Xaa)n-Peptide, worin Xaa eine α-Aminosäure ist, (n=0-10), entsprechende Derivate und deren Salze bzw. Aminosäure (Xaa)-amide, entsprechende Derivate und deren Salze, wobei Xaa eine α-Aminosäure/Iminosäure bzw. ein α-Aminosäurederivat/iminosäurederivat, vorzugsweise N^{ε}-4-Nitrobenzyloxycarbonyl-L-Lysin, L-Prolin, L-Tryptophan, L-Isoleucin, L-Valin ist und als Amidstruktur cyclische Amine, z.B. Pyrrolidin, Piperidin, Thiazolidin und deren Derivate fungieren. Derartige Verbindungen und deren Herstellung wurden in einem früheren Patent beschrieben (K. Neubert et al. DD296075A5).

Die Inhibitoren werden simultan mit bekannten Trägerstoffen verabreicht. Die Verabreichung erfolgt einerseits als topische Applikation in Form von z.B. Cremes, Salben, Pasten, Gelen, Lösungen, Sprays, Liposomen, Schüttelmixturen, Hydrokolloidverbänden bzw. anderen dermatologischen Grundlagen/Vehikeln einschließlich instillativer Applikation und andererseits als systemische Applikation zur oralen, transdermalen, intravenösen, subcutanen, intracutanen, intramuskulären, rektalen, vaginalen, sublingualen Anwendung in geeigneten Rezepturen bzw. in geeigneter Galenik mit an sich bekannten Träger-, Hilfs- und/oder Züsatzstoffen.

Für alle allergischen Reaktionen vom Typ I wie Asthma bronchiale oder Heuschnupfen gilt, dass eine Aktivierung, Proliferation und Zytokinproduktion (besonders IL-4) von Immunzellen, insbesondere von T_{H2}-Zellen, dem Krankheitsprozess zugrunde liegen [D.D. Corry et al.: Induction and regulation of the IgE response. Nature 1999; 402: B18-B23].

Es ist gezeigt worden, dass im Prozess der Aktivierung und klonalen Expansion von Immunzellen, insbesondere von T-Lymphozyten, membranständige Peptidasen wie DP IV oder APN eine Schlüsselrolle spielen [Fleischer B: CD26 a surface protease involved in T-cell activation. Immunology Today 1994; 15:180-184; Lendeckel U et al.: Role of alanyl aminopeptidase in growth and function of human T cells. International Journal of Molecular Medicine 1999; 4:17-27; Riemann D et al.: CD13 - not just a marker in leukemia typing. Immunology Today 1999; 20:83-88]. Verschiedene Funktionen Mitogen-stimulierter mononukleärer Zellen (MNZ) oder angereicherter T-Lymphozyten wie DNS-Synthese, Produktion und Sekretion von immunstimulierenden Zytokinen (IL-2, IL-6, IL-12, IFN-γ) und Helferfunktionen für B-Zellen (IgG- und IgM-Synthese) können in Gegenwart von spezifischen Inhibitoren der DP IV oder der APN gehemmt werden [Schön E et al.: The dipeptidyl peptidase IV, a membrane enzyme involved in the proliferation of T lymphocytes. Biomed. Biochim. Acta 1985; 2: K9-K15; Schön E et al.: The role of dipeptidyl peptidase IV in human T lymphocyte activation. Inhibitors and antibodies against dipeptidyl peptidase IV suppress lymphocyte proliferation and immunoglobulin synthesis in vitro. Eur. J. Immunol. 1987; 17: 1821-1826; Reinhold D et al.: Inhibitors of dipeptidyl peptidase IV induce secretion of transforming growth factor β1 in PWM-stimulated PBMNC and T cells. Immunology 1997; 91: 354-360; Lendeckel U et al. : Induction of the membrane alanyl aminopeptidase gene and surface expression in human T-cells by mitogenic activation. Biochem. J. 1996; 319: 817-823; Kähne T et al.: Dipeptidyl peptidase IV: A cell surface peptidase involved in regulating T cell growth (Review). Int. J. Mol. Med. 1999; 4: 3-15; Lendeckel U et al.: Role of alanyl aminopeptidase in growth and function of human T cells (Review). Int. J. Mol. Med. 1999; 4: 17-27].

Auf der anderen Seite haben wissenschaftliche Erkenntnisse der letzten Jahre die allergische Reaktion vom Typ I als eine Erkrankung charakterisiert, bei der den T_{H2}-Lymphozyten eine entscheidende Rolle für die Enstehung und Chronifizierung der Erkrankung zukommt [D.D. Corry et al.: Induction and regulation of the IgE response. Nature 1999; 402: B18-B23. P.J. Barnes: Therapeutic strategies for allergic diseases. Nature 1999; 402: B31-B38].

IL-4 stellt ein Helferzytokin der B-Zell-Proliferation dar, stimuliert die Bildung von IgE und die Expression niedrigaffiner Fc-IgE-Rezeptoren. Darüber hinaus verstärkt IL-4 die Induktion von T_{H2}-Zellen selbst und kontrolliert die Proliferation und Aktivität von Eosinophilen und Mastzellen. Damit spielt es eine zentrale Rolle bei allergischen Reaktionen vom Typ I [D.P. Stites, A.I. Terr, T.G. Parslow: Medical Immunology. Appelton & Lange, Stamfort, CT, 1997].

Der Erfindung liegt ebenfalls der überraschende Befund zugrunde, dass die gleichzeitige Wirkung_ von Inhibitoren der Dipeptidylpeptidase IV und der Aminopeptidase N, die Proliferation (DNS-Synthese) und IL-4-Produktion mitogenstimulierter mononukleärer Zellen (MNZ) in einem Ausmaß hemmt, das durch die einzelne Applikation dieser Enzyminhibitoren - auch bei höherer Dosierung - nicht erreicht werden kann. Obgleich die genannten Inhibitoren letztendlich die gleichen Prozesse, nämlich die DNS-Synthese und damit die Proliferation sowie die IL-4-Produktion der T_{H2-}Zellen, beeinflussen, ist dieser Effekt bei einzelner Applikation der Inhibitoren wesentlich schwächer ausgeprägt und nicht dauerhaft. Wegen der funktionellen Überschneidung der enzymatischen Aktivitäten der genannten Enzyme resultiert, wie unsere Daten zeigen, eine additive bis superadditive Hemmwirkung auf DNS-Synthese und Proliferation aus der gleichzeitigen Hemmung beider Enzyme.

Unsere Erfindung zeigt, dass zur Therapie allergischer Reaktionen vom Typ I für deren Entstehung die Proliferation und die Aktivierung von T_{H2}-Lymphozyten eine zentrale Bedeutung hat, die gleichzeitige Applikation von Hemmstoffen der DP IV und der APN bzw. entsprechender Zubereitungen und Darreichungsformen daraus geeignet sind.

Im einzelnen liegen der Erfindung die Befunde zugrunde, dass die DNS-Synthese und die IL-4-Produktion von MNZ durch die simultane Administration von Inhibitoren der Dipeptidylpeptidase IV sowie von Enzymen mit gleicher Substratspezifität (DPIV-analoger Enzymaktivität) und Aminopeptidase N sowie von Enzymen mit gleicher Substratspezifität (APN-analoger Enzymaktivität) in additiver bis superadditiver Weise inhibiert wird.

Die Applikation von Enzyminhibitoren stellt bei den genannten Erkrankungen eine neuartige Methode und ergänzende Therapieform dar.

Die erfindungsgemäß applizierten Inhibitoren der Dipeptidylpeptidase IV und der Aminopeptidase N können in pharmazeutisch anwendbaren Formulierungskomplexen als Inhibitoren, Substrate, Pseudosubstrate, inhibitorisch wirkende Peptide und Peptidderivate dieses Enzyms zur Anwendung kommen. Bevorzugte Effektoren sind beispielsweise für die DP IV Xaa-Pro-Dipeptide, worin xaa eine α-Aminosäure bzw. ein seitenkettengeschütztes Derivat ist, entsprechende Derivate, vorzugsweise Dipeptidphosphonsäurediarylester, Dipeptidboronsäuren (z.B. Pro-boro-Pro) und deren Salze, Xaa-Xaa-(Trp)-Pro-(Xaa)n-Peptide, worin xaa eine α-Aminosäure ist, (n=0-10), entsprechende Derivate und deren Salze bzw. Aminosäure (Xaa)-amide, entsprechende Derivate und deren Salze, wobei Xaa eine α-Aminösäure/Iminosäure bzw. ein α-Aminosäurederivat/Iminosäurederivat, vorzugsweise N^{ε}-4-Nitrobenzyloxycarbonyl-L-Lysin, L-Prolin, L-Tryptophan, L-Isoleucin, L-Valin ist und als Amidstruktur cyclische Amine, z.B. Pyrrolidin, Piperidin, Thiazolidin und deren Derivate fungieren. Derartige Verbindungen und deren Herstellung wurden in einem früheren Patent beschrieben (K. Neubert et al. DD296075A5).

Bevorzugte Inhibitoren für die Alanyl-Aminopeptidase sind Bestatin (Ubenimex), Actinonin, Probestin, Phebestin, RB3014 oder Leuhistin, Amastatin, β-Aminothiole α-Aminophosphinsäuren, α-Aminophosphinsäurederivate, vorzügsweise D-Phe-Ψ[PO(OH)-CH₂]-Phe-Phe und deren Salze.

Die Inhibitoren werden simultan mit bekannten Trägerstoffen verabreicht. Die Verabreichung erfolgt einerseits als topische Applikation in Form von z.B. Cremes, Salben, Pasten, Gelen, Lösungen, Sprays, Liposomen, Schüttelmixturen, Hydrokolloidverbänden bzw. anderen dermatologischen Grundlagen/Vehikeln einschließlich instillativer Applikation und andererseits als systemische Applikation zur oralen, transdermalen, intravenösen, subcutanen, intracutanen, intramuskulären rektalen, vaginalen, sublingualen Anwendung in geeigneten Rezepfuren bzw. in geeigneter Galenik. zusammen mit an sich bekannten Träger-, Hilfs- und/oder Zusatzstoffen.

Eine Reihe dermatologischer Erkrankungen gehen mit follikulären und epidermalen Hyperkeratosen und einer verstärkten Keratinozytenproliferation einher. Zu ihnen gehören sowohl entzündliche und nicht entzündliche epidermale Hyperproliferations-Zustände (z. B. congenitale Ichthyosen und Psoriasis), benigne und maligne umschriebene epidermale clonale Expansionen (z. B. Warzen, Condylome, aktinische Keratosen/Präcancerosen), benigne und maligne follikuläre Hyperproliferations-Zustände (z. B. Keratosis follikularis) als auch benigne und maligne epitheliale Adnextumoren und primäre und reaktive Nagelzellhyperproliferationen. Eine Detailinformation dazu ist in Tabelle 1 beigefügt.

Peptidasen wie die Dipeptidylpetidase IV und die Aminopeptidase N oder ähnlich wirkende Enzyme sind für die Regulation bzw. Modulation von Wechselwirkungen zwischen Zellen besonders interessant, da sie u. a. als Ektoenzyme in der Plasmamembran der Zellen lokalisiert sind, Interaktionen mit anderen extrazellulären Strukturen eingehen, peptiderge Botenstoffe durch enzymkatalysierte Hydrolyse aktivieren bzw. inaktivieren und dadurch wichtig für die Zell-Zell-Kommunikation sind [Yaron A, et al.: Proline-dependent structural and biological properties of peptides and proteins. Crit Rev Biochem Mol Biol 1993;28:31-81; Vanhoof G, et al.: Proline motifs in peptides and their biological processing. FASEB J 1995;9:736-744].

Es ist bereits bekannt, daß die Behandlung von Autoimmunerkrankungen und Transplantatabstoßung durch Hemmung der auf Immunzellen lokalisierten Dipeptidylpetidase IV mit Hilfe von synthetischen Inhibitoren möglich ist (z. B. EP764151 A1, WO09529691, EP731789 A1, EP528858).

Der Erfindung liegt der überraschende Befund zugrunde, dass die gleichzeitige Wirkung von Inhibitoren der auf bzw. in Keratinozyten exprimierten Dipeptidylpeptidase IV/CD26 und Aminopeptidase N/CD13 oder ähnlicher Enzyme, die Proliferation (DNS-Synthese) dieser Zellen in einem Ausmaß hemmt, das durch die einzelne Applikation dieser Enzyminhibitoren bei der gegebenen Dosierung nicht erreicht werden kann. Obgleich die genannten Inhibitoren letztendlich die gleichen Prozesse, nämlich die DNS-Synthese und damit die Proliferation der Keratinozyten, beeinflussen, ist dieser Effekt bei einzelner Applikation der Inhibitoren schwächer ausgeprägt und nicht dauerhaft. Wegen der funktionellen Überschneidung der enzymatischen Aktivitäten der genannten Enzyme resultiert, wie unsere Daten zeigen, eine additive und bei niedrigeren Konzentrationen eine additive bis superadditive Hemmwirkung auf DNS-Synthese und Proliferation aus der gleichzeitigen Hemmung beider Enzyme.

Unsere Erfindung zeigt, dass zur Therapie und zur Prävention von sowohl entzündlichen und nicht entzündlichen epidermalen Hyperproliferationszuständen (z. B. congenitale Ichthyosen und Psoriasis), benignen und malignen umschriebenen epidermalen clonalen Expansionen (z. B. Warzen, Condylome, aktinische Keratosen/ Präcancerosen), benignen und malignen follikulären Hyperproliferationszuständen (z. B. Keratosis follikularis) als auch benignen und malignen epithelialen Adnextumoren und primären und reaktiven Nagelzellhyperproliferationen für deren Enstehung die Proliferation und die Aktivierung von epidermalen und follikulären Keratinozyten sowie von Keratinozyten der Übergangsschleimhautzone eine zentrale Bedeutung hat, die gleichzeitige Applikation von Hemmstoffen der DP IV und der APN oder ähnlicher Enzyme bzw. entsprechender Zubereitungen und Darreichungsformen daraus geeignet sind.

Neben Keratinozyten spielen auch T-Lymphozyten bei entzündlichen Erkrankungen der Haut, insbesondere bei Autoimmunerkrankungen wie der Psoriasis, eine zentrale Rolle. T-Zellen exprimieren wie Keratinozyten die hier behandelten Peptidasen DP IV und APN. Daraus folgt, dass der therapeutische Effekt, der hier für den Zelltyp Keratinozyten beansprucht bzw. geschützt wird, durch die Beeinflussung der T-Zellen weiter verstärkt wird (siehe Patentanmeldung AZ 10025464.0; Kombinierte Verwendung von Enzyminhibitoren und pharmazeutischen Zubereitungen daraus zur Therapie von Autoimmunerkrankungen wie Rheumatoide Arthritis, Lupus erythematodes, Multiple Sklerose, Insulin-abhängiger Diabetes mellitus (IDDM), Morbus Crohn, Colitis Ulcerosa, Psoriasis, Neurodermitis, Glomerulonephritis, interstitielle Nephritis, Vaskulitis, autoimmune Schilddrüsenerkrankungen oder autoimmunhämolytische Anämie, sowie bei Transplantation und Tumorerkrankungen).

Im einzelnen liegen der Erfindung die Befunde zugrunde, dass die DNS-Synthese von HaCaT-Keratinozyten durch die simultane Administration von Inhibitoren der Dipeptidylpeptidase IV sowie von Enzymen mit gleicher Sübstratspezifität (DP IV-analoger Enzymaktivität) und der Aminopeptidase N sowie von Enzymen mit gleicher Sübstratspezifität (APN-analoger Enzymaktivität) in additiver und bei kleineren Konzentrationen in superadditiver Weise inhibiert wird.

Die oben genannten Erkrankungen werden bisher topisch mit antiproliferativen und differenzierenden Substanzen (Salizylsäure, Harnstoff, endogene und synthetische Retinoide, Vitamin D3-Derivate, Kortikosteroide) sowie systemisch mit z. T. immunsuppressiven und antiproliferativen Präparaten (z. B. Cyclosporin A, Kortikosteroide, Retinoide) behandelt. Insbesondere bei der systemischen Anwendung treten häufig unerwünschte Nebenwirkungen auf. Der kombinierte Einsatz von DP IV- und APN-Inhibitoren würde bei den genannten Erkrankungen eine neuartige, vorraussichtlich sehr effektive, möglicherweise kostengünstige Therapieform und einen wertvollen alternativen Bestandteil der bestehenden Therapiekonzepte darstellen.

Die erfindungsgemäß applizierten Inhibitoren der Dipeptidylpeptidase IV sowie von Enymen mit gleicher Substratspezifität (DP IV-analoger Enzymaktivität) und der Aminopeptidase N oder von Enzymen gleicher Substratspezifität (APN-analoger Enzymaktivität) können in pharmazeutisch anwendbaren Formulierungskomplexen als Inhibitoren, Substrate, Pseudosubstrate, inhibitorisch wirkende Peptide und Peptidderivate dieser Enzyme zur Anwendung kommen. Bevorzugte Inhibitoren sind beispielsweise für die DP IV Xaa-Pro-Dipeptide, entsprechende Derivate, vorzugsweise Dipeptidphosphonsäurediarylester, Dipeptidboronsäuren (z. B. Pro-boro-Pro) und deren Salze, Xaa-Xaa-(Trp)-Pro-(Xaa)n-Peptide (n=0 bis 10), entsprechende Derivate und deren Salze bzw. Aminosäure (Xaa)-amide, entsprechende Derivate und deren Salze, wobei Xaa eine α-Aminosäure/Iminosäure bzw. ein α-Aminosäurederivat/Iminosäurederivat, vorzugsweise N'-4-Nitrobenzyloxycarbonyl-L-Lysin, L-Isoleucin, L-Valin, L-Tryptophan, L-Prolin ist und als Amidstruktur cyclische Amine, z.B. Pyrrolidin, Piperidin, Thiazolidin und deren Derivate fungieren. Derartige Verbindungen und deren Herstellung wurden in einem früheren Patent beschrieben (K. Neubert et al. DD296075A5).

Bevorzugte Inhibitoren für die Alanyl-Aminopeptidase sind Bestatin (Ubenimex), Actinonin, Probestin, Phebestin, RB3014 oder Leuhistin.

Die Inhibitoren werden simultan mit bekannten Trägerstoffen verabreicht. Die Verabreichung erfolgt einerseits als topische Applikation in Form von z.B. Cremes, Salben, Pasten, Gelen, Lösungen, Sprays, Liposomen, Schüttelmixturen, Hydrokolloidverbänden, Pflaster und ähnlichen neuen Trägersubstraten, Jet-Injektionen bzw. anderen dermatologischen Grundlagen/Vehikeln einschließlich instillativer Applikation und andererseits als systemische Applikation zur oralen, transdermalen, intravenösen, subcutanen, intracutanen, intramuskulären Anwendung in geeigneten Rezepturen bzw. in geeigneter Galenik.

**Tabelle 1:**

| epidermale Hyperproliferationszustände | |
|---|---|
| z. B. nicht entzündlich | z. B. entzündlich |
| - congenitale Ichthyosen | |
| - acquirierte Ichthyosen (paraneoplast.) | - Lichen ruber und Subtypen |
| - Palmoplantarkeratosen | - Keratosis lichenoides |
| - congenital | - Lichen simplex chronicus + reaktive liche-noide |
| - erworben/paraneoplast. | Hyperproliferationen (z. B. atopische Dermatitis |
| - M. Darier | Lichenoide Reaktionen bei GvHD |
| - Epidermale Naevi | - ILVEN-Naevus |
| - Cutis rhomboidalis nuchae | - Lupus erythematodes chron. disc./SCLE/SLE |
| - Acanthosis nigricans | - Pityriasis rubra pilaris |
| - Pachydermie | - M. Grover |
| | - Vitiligo |
| | - mit Hyperproliferation von Keratinozyten einhergehende Erythrodermien |

| umschriebene epidermale clonale Expansion | |
|---|---|
| benigne | maligne |
| - HPV-assoziiert (Warzen/Condylome) | - HPV-assoziierte Tumoren |
| - seborrhoische Keratosen | - aktinische Keratosen/Präcancerosen |
| - Hidroakanthome/Porome | - M. Bowen + Bowen-CA |
| - Epidermalcysten | - M. Paget + Paget-CA |
| - Milien | - Plattenepithel-CA |
| - M. Gottron | - Merkelcell-CA |

| follikuläre Hyperproliferationszustände | |
|---|---|
| benigne | maligne |
| - Keratosis follikularis | - Haarfollikelzelltumoren |
| | - Proliferierende Trichilemmalcysten |
| - Ulerythema ophryogenes | - Mischtumoren |
| - Hypertrichosen | |
| - Trichilemmalcysten | |

| epitheliale Adnextumoren | |
|---|---|
| benigne | maligne |
| - Porom | - ekkrine/apokrine CA's und Subtypen |
| - syringoductale Tumoren | |
| - Hidraadenome | |
| - Spiradenome | |
| - Cylindrome | |

| primäre und reaktive Nagelzellhyperproliferation | |
|---|---|
| | nicht infektiös |
| - congenital | erworben |
| z. B. Pachyonychien | infektiös bei Mykosen |

Die Erfindung wird anhand der nachfolgenden Ausführungsbeispiele näher erläutert, ohne auf diese beschränkt zu sein.

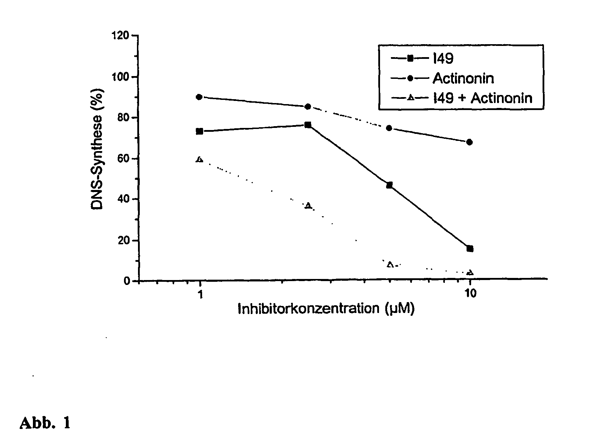

In den Beispielen wird auf Abbildungen 1 bis 14 Bezug genommen. Diese zeigen:
**Abb. 1**: Synergistischer und dosisabhängiger Effekt von Inhibitoren der DP IV (I49) und der Aminopeptidase N (Actinonin) auf die DNS-Synthese humaner T-Lymphozyten. Humane periphere T-Zellen wurden über drei Tage mit den angegebenen Konzentrationen der Inhibitoren inkubiert. Anschließend wurde dem Kulturmedium ³[H]-Methyl-Thymidin zugesetzt und nach weiteren 6 Stunden die in die DNS eingebaute Menge an ³[H]-Thymidin gemessen.

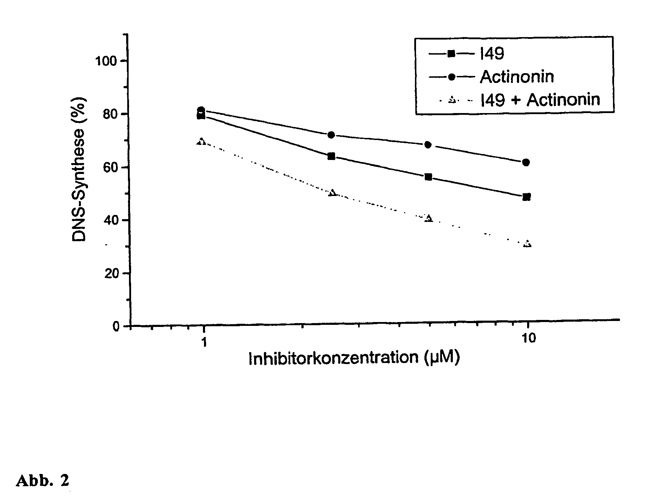

**Abb. 2**: Synergistischer und dosisabhängiger Effekt von Inhibitoren der DP IV (I49) und der APN (Actinonin) auf die DNS-Synthese humaner mononukleärer Zellen (MNZ). Humane MNZ wurden über drei Tage mit den angegebenen Konzentrationen der Inhibitoren inkubiert. Anschließend wurde dem Kulturmedium ³[H]-Methyl-Thymidin zugesetzt und nach weiteren 6 Stunden die in die DNS eingebaute Menge an ³[H]-Thymidin gemessen.

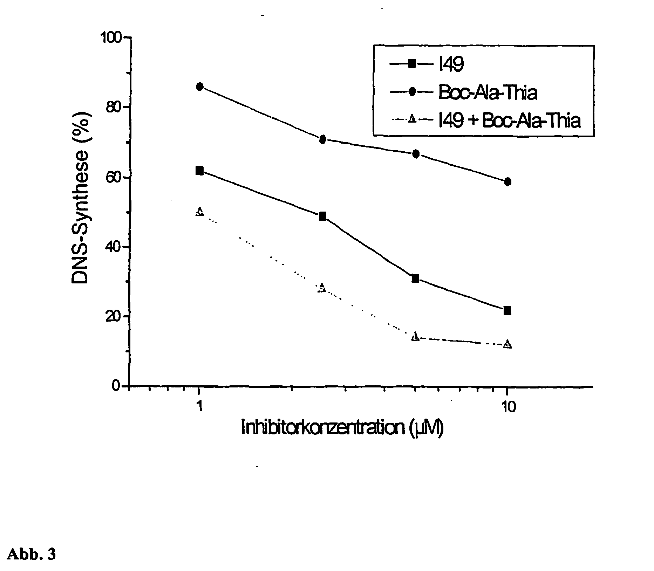

**Abb. 3****:** Synergistischer und dosisabhängiger Effekt von Inhibitoren der DP IV (I49) und der Prolyloligopeptidase (Boc-Ala-Thia) auf die DNS-Synthese humaner peripherer T-Lymphozyten. Humane T-Zellen wurden über drei Tage mit den angegebenen Konzentrationen der Inhibitoren inkubiert. Anschließend wurde dem Kulturmedium ³[H]-Methyl-Thymidin zugesetzt und nach weiteren 6 Stunden die in die DNS eingebaute Menge an ³[H]-Thymidin gemessen.

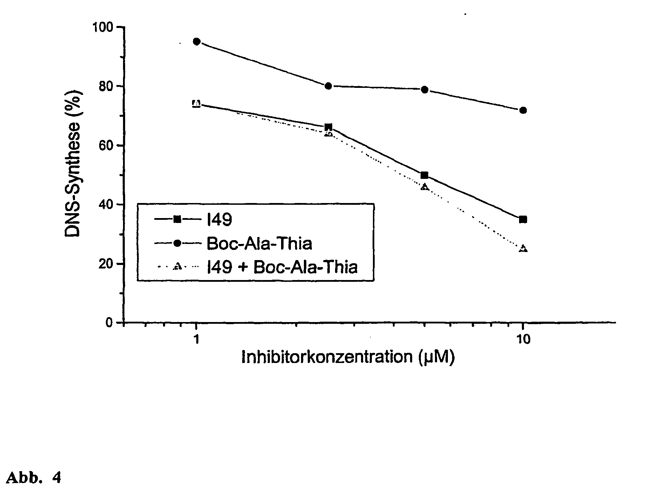

**Abb. 4****:** Verstärkter und dosisabhängiger Effekt von Inhibitoren der DP IV (I49) und der Prolyloligopeptidase (Boc-Ala-Thia) auf die DNS-Synthese humaner mononukleärer Zellen (MNZ). Humane MNZ wurden über drei Tage mit den angegebenen Konzentrationen der Inhibitoren inkubiert. Anschließend wurde dem Kulturmedium ³[M-Methyl-Thymidin zugesetzt und nach weiteren 6 Stunden die in die DNS eingebaute Menge an ³[H]-Thymidin gemessen.

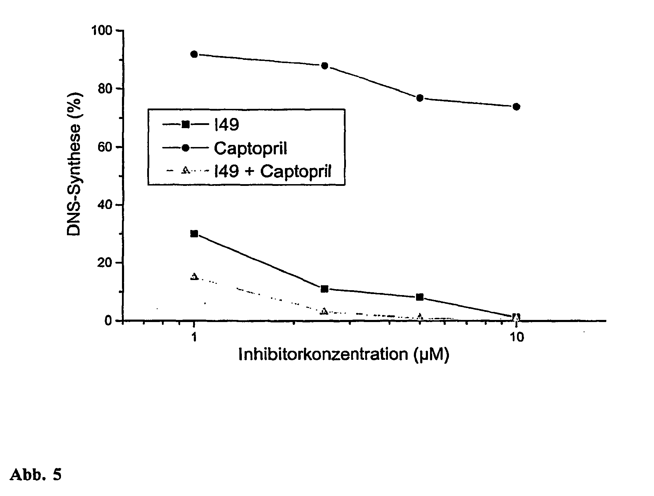

**Abb. 5****:** Synergistischer und dosisabhängiger Effekt von Inhibitoren der DP IV (I49) und des Angiotensin-konvertierenden Enzyms (Captopril) auf die DNS-Synthese humaner peripherer T-Lymphozyten. Humane T-Zellen wurden über drei Tage mit den angegebenen Konzentrationen der Inhibitoren inkubiert. Anschließend wurde dem Kulturmedium ³[H]-Methyl-Thymidin zugesetzt und nach weiteren 6 Stunden die in die DNS eingebaute Menge an ³[H]-Thymidin gemessen.

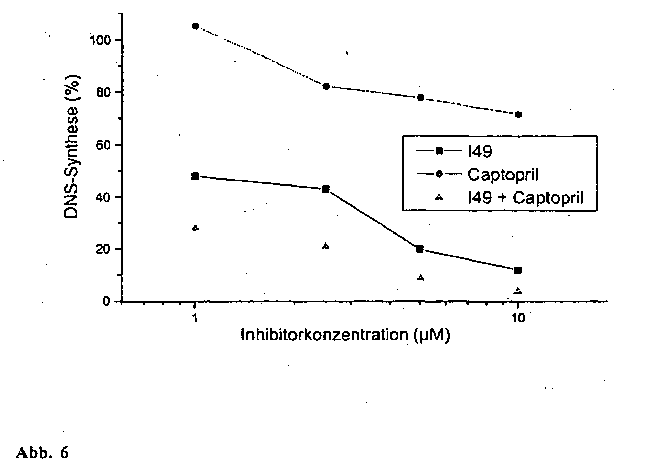

**Abb. 6****:** Synergistischer und dosisabhängiger Effekt von Inhibitoren der DP IV (I49) und des Angiotensin-konvertierenden Enzyms (Captopril) auf die DNS-Synthese humaner mononukleärer Zellen (MNZ). Humane MNZ wurden über drei Tage mit den angegebenen Konzentrationen der Inhibitoren inkubiert. Anschließend wurde dem Kulturmedium ³[H]-Methyl-Thymidin zugesetzt und nach weiteren 6 Stunden die in die DNS eingebaute Menge an ³[H]-Thymidin gemessen.

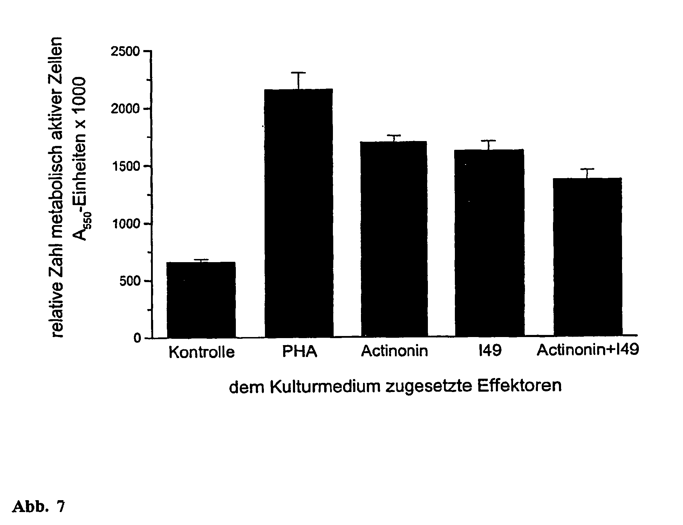

**Abb. 7****:** Die MNZ wurden über einen Zeitraum von 72 h ohne Zusatz (Kontrolle), mit dem mitogenen Lektin Phytohämagglutinin (PHA) bzw. mit PHA und den angegebenen Inhibitoren inkubiert. Anschließend erfolgte die Bestimmung der Zahl metabolisch aktiver Zellen unter Verwendung des kommerziell verfügbaren WST-1 Zell-Proliferations-Assays (Takara Inc.) nach Angaben des Herstellers.

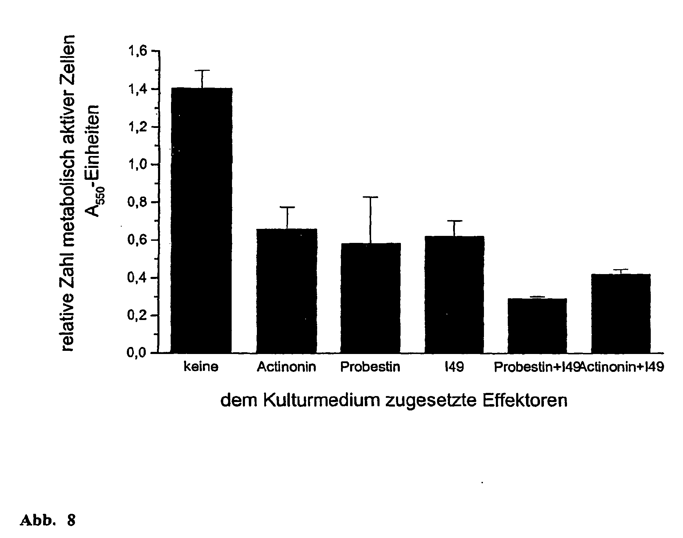

**Abb. 8****:** Die KARPAS-299-Zellen wurden über einen Zeitraum von 72 h ohne Zusatz (Kontrolle) bzw. in Gegenwart der angegebenen Inhibitoren einzeln sowie in Kombination inkubiert. Anschließend erfolgte die Bestimmung der Zahl metabolisch aktiver Zellen unter Verwendung des kommerziell verfügbaren WST-1 Zell-Proliferations-Assays (Takara Inc.) nach Angaben des Herstellers.

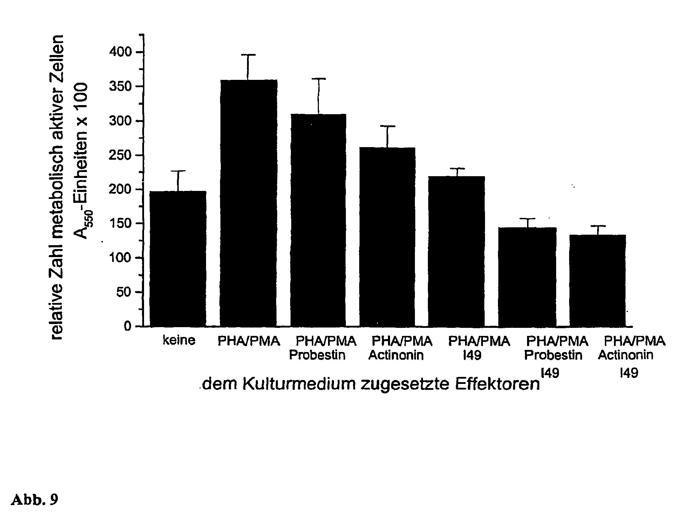

**Abb. 9****:** Die T-Zellen wurden mit Ausnahme der unbehandelten Kontrolle durch Zugabe zum Kulturmedium von Phytohämagglutinin und Phorbol-12-myristat-13-acetat aktiviert und über einen Zeitraum von 72 h in Gegenwart der angegebenen Inhibitoren einzeln sowie in Kombination inkubiert. Anschließend erfolgte die Bestimmung der Zahl metabolisch aktiver Zellen unter Verwendung des kommerziell verfügbaren WST-1 Zell-Proliferations-Assays (Takara Inc.) nach Angaben des Herstellers.

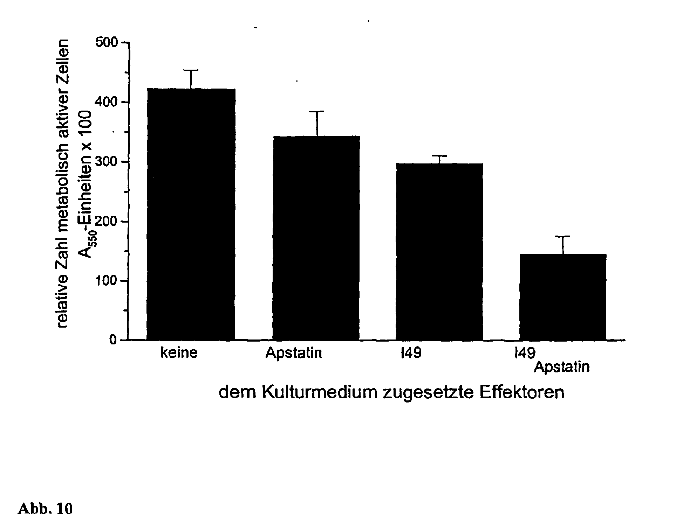

**Abb. 10****:** Die mononukleären Zellen (MNZ) wurden über einen Zeitraum von 72 h in Gegenwart der angegebenen Inhibitoren einzeln sowie in Kombination inkubiert. Anschließend erfolgte die Bestimmung der Zahl metabolisch aktiver Zellen unter Verwendung des kommerziell verfügbaren WST-1 Zell-Proliferations-Assays (Takara Inc.) nach Angaben des Herstellers.

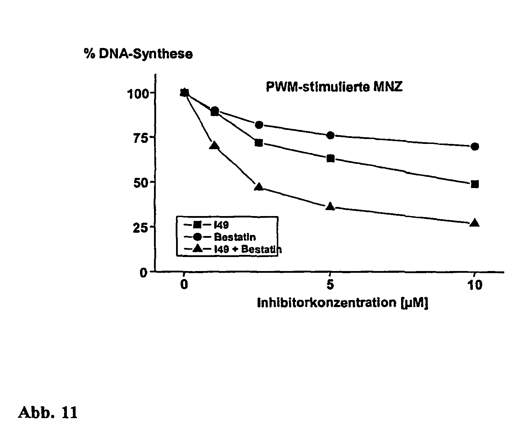

**Abb. 11****:** Synergistischer und dosisabhängiger Effekt von Inhibitoren der DP IV (I49) und der Aminopeptidase N (Bestatin) auf die DNS-Synthese humaner PWM-stimulierter MNZ. Humane periphere MNZ wurden über drei Tage mit PWM (2 µg/ml) und den angegebenen Konzentrationen der Inhibitoren inkubiert. Anschließend wurde dem Kulturmedium ³[H]-Methyl-Thymidin zugesetzt und nach weiteren 6 Stunden die in die DNS eingebaute Menge an ³[H]-Thymidin gemessen.

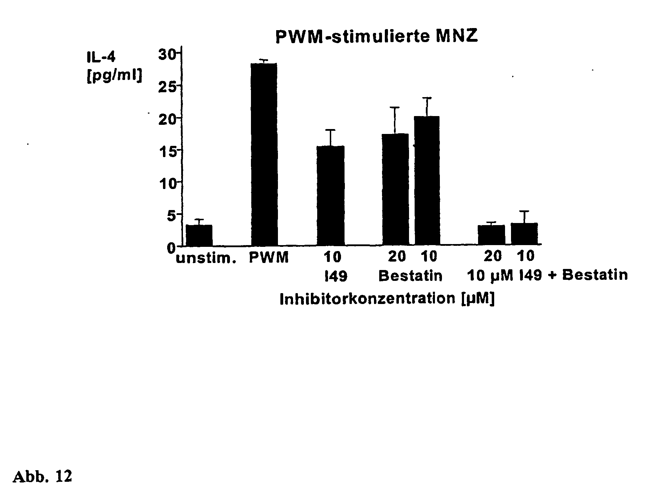

**Abb. 12****:** Synergistischer und dosisabhängiger Effekt von Inhibitoren der DP IV (I49) und der Aminopeptidase N (Bestatin) auf die IL-4-Produktion humaner, PWM-stimulierter MNZ. Humane periphere MNZ wurden über 48 h mit PWM (2 µg/ml) und den angegebenen Konzentrationen der Inhibitoren inkubiert. Anschließend wurden mittels IL-4-ELISA die Konzentrationen von IL-4 in den entsprechenden Kulturüberständen gemessen.

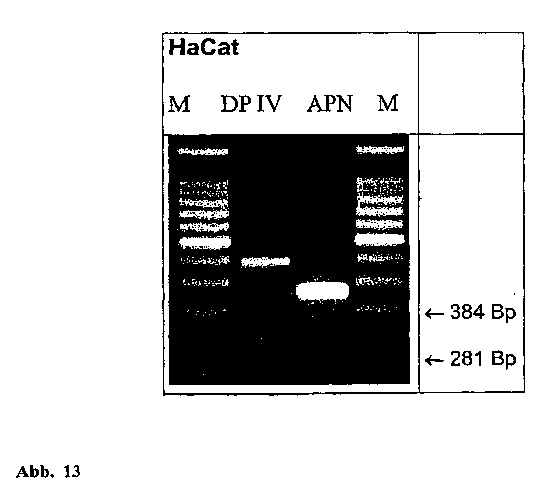

**Abb. 13****:** Nachweis der mRNA-Expression von DP IV und APN auf HaCaT-Keratinozyten mittels RT-PCR

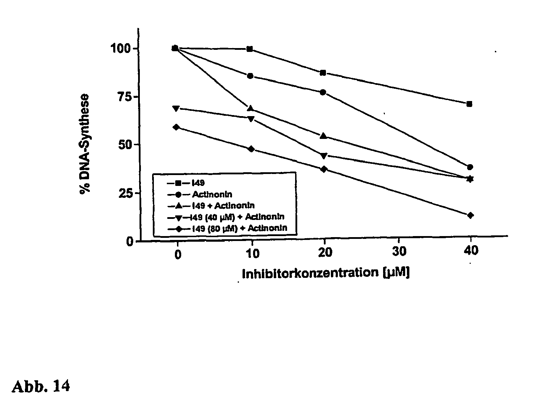

**Abb.14****:** Synergistischer und dosisabhängiger Effekt von Inhibitoren der DP IV (I49) und der Aminopeptidase N (Actinonin) auf die DNS-Synthese humaner HaCaT-Keratinozyten. Die Zellen wurden über 48 Stunden mit den angegebenen Konzentrationen der Inhibitoren inkubiert. Anschließend wurde dem Kulturmedium 3[H]-Methyl-Thymidin zugesetzt und nach weiteren 6 Stunden die in die DNS eingebaute Menge an 3[H]-Thymidin gemessen.

### Beispiel 1

### Inhibierung der DNS-Synthese von humanen T-Lymphozyten durch Inkubation mit synthetischen Inhibitoren der DP IV und der APN

Unsere Untersuchungen zeigten, dass die DNS-Synthese humaner peripherer T-Lymphozyten durch die simultane Administration von Inhibitoren der DP IV (Lys[Z(NO₂)]-thiazolidid = I49) und APN (Actinonin) in additiver bis superadditiver Weise gehemmt wird. Die T-Zellen wurden 72 h in Gegenwart der genannten Inhibitoren inkubiert und anschließend über die Messung der ³[H]-Thymidin-Inkorporation die DNS-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 1 (Seite 1/14) zeigt die dosisabhängige, additive bis superadditive Hemmung der DNS-Synthese.

### Beispiel 2

### Inhibierung der DNS-Synthese von humanen peripheren mononukleären Zellen durch Inkubation mit synthetischen Inhibitoren der DP IV und der APN

Unsere Untersuchungen zeigten, dass die DNS-Synthese humaner peripherer mononukleärer Zellen (MNZ) durch die simultane Administration von Inhibitoren der DP IV (Lys[Z(NO₂)]-thiazolidid = I49) und APN (Actinonin) in additiver bis superadditiver Weise gehemmt wird. Die MNZ wurden 72 h in Gegenwart der genannten Inhibitoren inkubiert und anschließend über die Messung der ³[H]-Thymidin-Inkorporation die DNS-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 2 (Seite 2/14) zeigt die dosisabhängige, additive bis superadditive Hemmung der DNS-Synthese.

### Vergleichs-Beispiel 3, das zum besseren Verständnis der Erfindung dient

### Inhibierung der DNS-Synthese von humanen T-Lymphozyten durch Inkubation mit synthetischen Inhibitoren der DP IV und der POP

Unsere Untersuchungen zeigten, dass die DNS-Synthese humaner T-Lymphozyten durch die simultane Administration von Inhibitoren der DP IV (Lys[Z(NO₂)]-thiazolidid = I49) und der Prolyloligopeptidase (Boc-Ala-Thiazolidid) in additiver bis superadditiver Weise gehemmt wird. Die T-Zellen wurden 72 h in Gegenwart der genannten Inhibitoren inkubiert und anschließend über die Messung der ³[H]-Thymidin-Inkorporation die DNS-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 3 (Seite 3/14) zeigt die dosisabhängige, additive bis superadditive Hemmung der DNS-Synthese.

### Vergleichs-Beispiel 4, das zum besseren Verständnis der Erfindung dient

### Inhibierung der DNS-Synthese von humanen peripheren mononukleären Zellen durch Inkubation mit synthetischen Inhibitoren der DP IV und der POP

Unsere Untersuchungen zeigten, dass die DNS-Synthese humaner peripherer mononukleärer Zellen (MNZ) durch die simultane Administration von Inhibitoren der DP IV (Lys[Z(NO₂)]-thiazolidid = I49) und der Prolyloligopeptidase (Boc-Ala-Thiazolidid) verstärkt gehemmt wird. Die MNZ wurden 72 h in Gegenwart der genannten Inhibitoren inkubiert und anschließend über die Messung der ³[H]-Thymidin-Inkorporation die DNS-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 4 (Seite 4/14) zeigt die dosisabhängige, verstärkte Hemmung der DNS-Synthese.

### Vergleichs-Beispiel 5, das zum besseren Verständnis der Erfindung dient

### Inhibierung der DNS-Synthese von humanen T-Lymphozyten durch Inkubation mit synthetischen Inhibitoren der DP IV und des ACE

Unsere Untersuchungen zeigten, dass die DNS-Synthese humaner T-Lymphozyten durch die simultane Administration von Inhibitoren der DP IV (Lys[Z(NO₂)]-thiazolidid = I49) und des Angiotensin-konvertierenden Enzyms (Captopril) in additiver bis superadditiver Weise gehemmt wird. Die T-Zellen wurden 72 h in Gegenwart der genannten Inhibitoren inkubiert und anschließend über die Messung der ³[H]-Thymidin-Inkorporation die DNS-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 5 (Seite 5/14) zeigt die dosisabhängige, additive bis superadditive Hemmung der DNS-Synthese.

### Vergleichs-Beispiel 6, das zum besseren Verständnis der Erfindung dient

### Inhibierung der DNS-Synthese von humanen peripheren mononukleären Zellen durch Inkubation mit synthetischen Inhibitoren der DP IV und des ACE

Unsere Untersuchungen zeigten, dass die DNS-Synthese humaner peripherer mononukleärer Zellen (MNZ) durch die simultane Administration von Inhibitoren der DP IV (Lys[Z(NO₂)]-thiazolidid = I49) und des Angiotensin-konvertierenden Enzyms (Captopril) in additiver bis superadditiver Weise gehemmt wird. Die MNZ wurden 72 h in Gegenwart der genannten Inhibitoren inkubiert und anschließend über die Messung der ³[H]-Thymidin-Inkorporation die DNS-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 6 (Seite 6/14) zeigt die dosisabhängige, additive bis superadditive Hemmung der DNS-Synthese.

### Beispiel 7

**Hemmung der Proliferation von humanen peripheren mononukleären Zellen (MNZ) durch die einzelne und gleichzeitige Gabe von Hemmstoffen der DP IV (I49 = Lys[Z(NO₂)]-thiazolidid) und APN (Actinonin). (****Abbildung 7****: Seite 7/14)**

### Beispiel 8

**Hemmung der Proliferation der humanen T-Zelllinie KARPAS-299 durch die einzelne und gleichzeitige Gabe von Hemmstoffen der DP IV (I49 = Lys[Z(NO₂)]-thiazolidid) und APN (Actinonin und Probestin).** (Abbildung 8: Seite 8/14)

### Beispiel 9

**Hemmung der Proliferation aktivierter, humaner peripherer T-Zellen durch die einzelne bzw. gleichzeitige Gabe von Hemmstoffen der DP IV (I49 = Lys[Z(NO₂)]-thiazolidid) und APN (Actinonin und Probestin).** (Abbildung 9: Seite 9/14)

### Vergleichs-Beispiel 10, das zum besseren Verständnis der Erfindung dient

**Hemmung der Proliferation PHA-aktivierter, humaner mononukleärer Zellen (MNZ) durch die einzelne bzw. gleichzeitige Gabe von Hemmstoffen der DP IV (I49 = Lys[Z(NO₂)]-thiazolidid) und der X-Pro-Aminopeptidase (APP) (Apstatin).** (Abbildung 10: Seite 10/14)

### Beispiel 11

### Inhibierung der DNS-Synthese Pokeweed-Mitogen (PWM)-stimulierter humaner mononukleärer Zellen (MNZ) des peripheren Blutes durch Inkubation mit synthetischen Inhibitoren der DP IV und der APN

Unsere Untersuchungen zeigen, dass die DNS-Synthese Pokeweed-Mitogen-stimulierter humaner MNZ des peripheren Blutes durch die simultane Administration von Inhibitoren der DP IV (Lys[Z(NO₂)]-thiazolidid = I49) und APN (Bestatin) in additiver Weise gehemmt wird. Die MNZ wurden 72 h in Gegenwart von Pokeweed-Mitogen und der genannten Inhibitoren inkubiert und anschließend über die Messung der ³[H]-Thymidin-Inkorporation die DNS-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 11 (Seite 11/14 zeigt die dosisabhängige, additive Hemmung der DNS-Synthese.

### Beispiel 12

### Inhibierung der IL-4-Produktion Pokeweed-Mitogen-stimulierter humaner mononukleärer Zellen des peripheren Blutes durch Inkubation mit synthetischen Inhibitoren der DP IV und der APN

Unsere Untersuchungen zeigen den interssanten Befund, dass die Produktion des für T_{H2}-Zellen charakteristischen Zytokins IL-4 von Pokeweed-Mitogen-stimulierten humanen mononukleären Zellen (MNZ) des peripheren Blutes durch die simultane Administration von Inhibitoren der DP IV (Lys[Z(NO₂)]-thiazolidid = I49) und der APN (Bestatin) in superadditiver Weise gehemmt wird. Die MNZ wurden 48 h in Gegenwart von Pokeweed-Mitogen und der genannten Inhibitoren inkubiert und anschließend mittels kommerzieller IL-4-Bestimmungs-Kits (ELISA) die Konzentrationen des IL-4 in den entsprechenden Kulturüberständen bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 12 (Seite 12/14) zeigt die dosisabhängige, superadditive Hemmung der IL-4-Produktion.

### Beispiel 13

### Inhibierung der DNS-Synthese humaner Keratinozyten (HaCaT-Zelllinie) durch Inkubation mit synthetischen Inhibitoren der DP IV und der APN

Unsere Untersuchungen zeigen, dass die DNS-Synthese humaner HaCaT-Keratinozyten durch die simultane Administration von Inhibitoren der DP IV (Lys[Z(NO₂)]-thiazolidid = I49) und APN (Actinonin) in additiver und bei kleineren Konzentrationen auch in superadditiver Weise gehemmt wird.

Die humane Keratinozytenzellinie HaCat, welche als Zellmodell für die Psoriasis akzeptiert ist, exprimiert DP IV und APN. Die Enzymaktivität der DP IV von vitalen Zellen beträgt 30,2 ± 5 pkat/10⁶ Zellen, die der APN beträgt 90 ± 4 pkat/10⁶ Zellen. Entsprechend ist die mRNA von APN und DP IV auf diesen Zellen nachweisbar (Abb. 13, Seite 13/14)).

HaCaT-Zellen wurden 48 h mit den oben genannten Inhibitoren inkubiert und anschließend über die Messung der ³[H]-Thymidin-Inkorporation die DNS-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 14 (Seite 14/14) zeigt die dosisabhängige Hemmung der DNS-Synthese.

## Patentansprüche

1. Verwendung von wenigstens einem Inhibitor, ausgewählt aus der Gruppe, die besteht aus Inhibitoren der Dipeptidylpeptidase IV (DP IV) und Inhibitoren von Enzymen mit gleicher Substratspezifität (DP IV-analoger Enzymaktivität),
ausgewählt aus der Gruppe, die besteht aus
- Xaa-Pro-Dipeptiden oder deren Salzen,
worin Xaa eine α-Aminosäure oder ein seitenkettengeschütztes Derivat ist;
- Xaa-Xaa-(Trp)-Pro-(Xaa)n-Peptiden oder deren Salzen,
worin Xaa eine α-Aminosäure ist und n für eine ganze Zahl von 0 bis 10 steht; und
- Aminosäure(Xaa)-amiden oder deren Salzen,
worin Xaa eine α-Aminosäure oder ein seitenkettengeschütztes Derivat ist, und als Amidstruktur cyclische Amine fungieren,
in Kombination mit wenigstens einem Inhibitor, ausgewählt aus der Gruppe, die besteht aus Inhibitoren der Alanyl-Aminopeptidase (Aminopeptidase N, APN) und Inhibitoren von Enzymen mit gleicher Substratspezifität (APN-analoger Enzymaktivität),
ausgewählt aus der Gruppe, die besteht aus Actinonin, Leuhistin, Phebestin, Amastatin, Bestatin, Probestin, RB3014, β-Aminothiolen, α-Aminophosphinsäuren und deren Salzen,
zur Herstellung eines Arzneimittels zur Vorbeugung und Therapie von allergischen Reaktionen vom Typ I.

2. Verwendung von wenigstens einem Inhibitor, ausgewählt aus der Gruppe, die besteht aus Inhibitoren der Dipeptidylpeptidase IV (DP IV) und Inhibitoren von Enzymen mit gleicher Substratspezifität (DP IV-analoger Enzymaktivität),
ausgewählt aus der Gruppe, die besteht aus
- Xaa-Pro-Dipeptiden oder deren Salzen,
worin Xaa eine α-Aminosäure oder ein seitenkettengeschütztes Derivat ist;
- Xaa-Xaa-(Trp)-Pro-(Xaa)n-Peptiden oder deren Salzen,
worin Xaa eine α-Aminosäure ist und n für eine ganze Zahl von 0 bis 10 steht; und
- Aminosäure(Xaa)-amiden oder deren Salzen,
worin Xaa eine α-Aminosäure oder ein seitenkettengeschütztes Derivat ist, und als Amidstruktur cyclische Amine fungieren,
in Kombination mit wenigstens einem Inhibitor, ausgewählt aus der Gruppe, die besteht aus Inhibitoren der Alanyl-Aminopeptidase (Aminopeptidase N, APN) und Inhibitoren von Enzymen mit gleicher Substratspezifität (APN-analoger Enzymaktivität),
ausgewählt aus der Gruppe, die besteht aus Actinonin, Leuhistin, Phebestin, Amastatin, Bestatin, Probestin, RB3014, β-Aminothiolen, α-Aminophosphinsäuren und deren Salzen,
zur Herstellung eines Arzneimittels zur Vorbeugung und Therapie von dermatologischen Erkrankungen, die mit follikulären und epidermalen Hyperkeratosen und einer verstärkten Keratinozytenproliferation einhergehen, ausgewählt aus der Gruppe, die besteht aus
entzündlichen epidermalen Hyperproliferationszuständen, ausgewählt aus der Gruppe, die besteht aus
Lichen ruber und Subtypen, Keratosis lichenoides, Lichen simplex chronicus und reaktive lichenoide Hyperproliferationen (z.B. atopische Dermatitis), lichenoide Reaktionen bei GvHD, ILVEN-Naevus, Lupus erythematodes chron. disc./SCLE/SLE, Pityriasis rubra pilaris, M. Grover, Vitiligo und mit Hyperproliferation von Keratinozyten einhergehende Erythrodermien;
nicht entzündlichen epidermalen Hyperproliferationszuständen;
benignen und malignen umschriebenen epidermalen clonalen Expansionen (z. B. Warzen, Condylome, aktinische Keratosen/Präcancerosen);
benignen und malignen follikulären Hyperproliferationszuständen, ausgewählt aus der Gruppe, die besteht aus Keratosis follikularis, Ulerythema ophryogenes, Hypertrichosen, Trichilemmalcysten, Haarfollikelzelltumoren, proliferierenden Trichilemmalcysten und Mischtumoren;
benignen und malignen epithelialen Adnextumoren; und
primären und reaktiven Nagelzellhyperproliferationen.

3. Verwendung nach Anspruch 1 oder Anspruch 2, worin das Xaa-Pro-Dipeptid ausgewählt ist aus der Gruppe, die besteht aus Dipeptidphosphonsäurediarylestern und Dipeptidboronsäuren.

4. Verwendung nach Anspruch 3, worin die Dipeptidboronsäure Pro-Boro-Pro ist.

5. Verwendung nach Anspruch 1 oder Anspruch 2, worin das Xaa des Aminosäure(Xaa)-amids ausgewählt ist aus der Gruppe, die besteht aus N^{ε}-4-Nitrobenzyloxycarbonyl-L-Lysin, L-Prolin, L-Tryptophan, L-Isoleucin und L-Valin.

6. Verwendung nach Anspruch 1 oder Anspruch 2, worin das cyclische Amin ausgewählt ist aus der Gruppe, die besteht aus Pyrrolidin, Piperidin und Thiazolidin.

7. Verwendung nach einem der Ansprüche 1 bis 6, worin das Amino-säure(Xaa)-amid ausgewählt ist aus der Gruppe, die besteht aus N^{ε}-4-Nitrobenzyl-oxycarbonyl-L-Lysin-thiazolidid, N^{ε}-4-Nitrobenzyloxycarbonyl-L-Lysin-pyrrolidid, N^{ε}-4-Nitrobenzyloxycarbonyl-L-Lysin-piperidid, N^{ε}-4-Nitrobenzyloxycarbonyl-L-Lysin-2-cyanothiazolidid, N^{ε}-4-Nitrobenzyloxycarbonyl-L-Lysin-2-cyanopyrrolidid und N^{ε}-4-Nitrobenzyloxycarbonyl-L-Lysin-2-cyanopiperidid.

8. Verwendung nach einem der Ansprüche 1 bis 7, worin der DP IV-Inhibitor Lys[Z(NO₂)]-thiazolidid ist, worin Lys für einen L-Lysin-Rest und Z(NO₂) für 4-Nitrobenzyloxycarbonyl steht.

9. Verwendung nach einem der Ansprüche 1 bis 8, worin die α-Aminophosphinsäure D-Phe-Ψ[PO(OH)-CH₂]-Phe-Phe ist.

10. Verwendung nach einem der Ansprüche 1 und 3 bis 9 zur Vorbeugung und Therapie von Asthma bronchiale oder Heuschnupfen.

11. Verwendung nach einem der Ansprüche 1 bis 10 in Kombination mit an sich bekannten Träger-, Hilfs- und/oder Zusatzstoffen.

12. Verwendung nach einem der Ansprüche 1 bis 11 für die systemische Anwendung zur oralen, transdermalen, intravenösen, subcutanen, intracutanen, intramuskulären, rektalen, vaginalen oder sublingualen Applikation.

13. Verwendung nach einem der Ansprüche 1 bis 11 für die topische Anwendung in Form von Cremes, Salben, Pasten, Gelen, Lösungen, Sprays, Liposomen, Schüttelmixturen, Hydrokolloidverbänden, Pflastern und ähnlichen Trägersubstraten, Jet-Injektionen bzw. anderen dermatologischen Grundlagen/Vehikeln, einschließlich instillativer Applikation.

14. Verwendung nach einem der Ansprüche 1 bis 13, worin der Inhibitor der Dipeptidylpeptidase IV (DP IV) oder der Inhibitor von Enzymen mit gleicher Substratspezifität (DP IV-analoger Enzymaktivität) und der Inhibitor der Alanyl-Aminopeptidase (Aminopeptidase N, APN) oder der Inhibitor von Enzymen mit gleicher Substratspezifität (APN-analoger Enzymaktivität) in räumlich getrennter Formulierung gleichzeitig oder zeitlich unmittelbar aufeinanderfolgend mit dem Ziel einer gemeinsamen Wirkung zu verabreichen sind.

## Claims

1. A use of at least one inhibitor, selected from the group, consisting of inhibitors of dipeptidyl peptidase IV (DP IV) and of inhibitors of enzymes having similar substrate specificity (DP IV analogous enzymatic activity), selected from the group, consisting of
- Xaa-Pro-dipeptides or salts thereof,
wherein Xaa is an α-amino acid or a side chain-protected derivative thereof;
- Xaa-Xaa-(Trp)-Pro-(Xaa)ₙ peptides or salts thereof,
wherein Xaa is an α-amino acid and n is an integer of 0 to 10; and
- amino acid(Xaa)-amides or salts thereof,
wherein Xaa is an α-amino acid or a side chain-protected derivative thereof and wherein the amide structure is a cyclic amine;
in combination with at least one inhibitor, selected from the group, consisting of inhibitors of alanyl-aminopeptidase (aminopeptidase N, APN) and of inhibitors of enzymes having a similar substrate specificity (APN-analogous enzymatic activity), selected from the group, consisting of actinonin, leuhistin, phebestin, amastatin, bestatin, probestin, RB3014, ß-aminothiols, α-aminophosphinic acids and salts thereof for the preparation of a pharmaceutical preparation for the prevention and therapy of allergic reactions of the type I.

2. A use of at least one inhibitor, selected from the group, consisting of inhibitors of dipeptidyl peptidase IV (DP IV) and of inhibitors of enzymes having similar substrate specificity (DP IV analogous enzymatic activity), selected from the group, consisting of
- Xaa-Pro-dipeptides or salts thereof,
wherein Xaa is an α-amino acid or a side chain-protected derivative thereof;
- Xaa-Xaa-(Trp)-Pro-(Xaa)ₙ peptides or salts thereof,
wherein Xaa is an α-amino acid and n is an integer of 0 to 10; and
- amino acid(Xaa)-amides or salts thereof,
wherein Xaa is an α-amino acid or a side chain-protected derivative thereof and wherein the amide structure is a cyclic amine;
in combination with at least one inhibitor, selected from the group, consisting of inhibitors of alanyl-aminopeptidase (aminopeptidase N, APN) and of inhibitors of enzymes having a similar substrate specificity (APN-analogous enzymatic activity), selected from the group, consisting of actinonin, leuhistin, phebestin, amastatin, bestatin, probestin, RB3014, ß-aminothiols, α-aminophosphinic acids and salts thereof for the preparation of a pharmaceutical preparation for the prevention and therapy of dermatological diseases, associated with follicular and epidermal hyperceratoses and an enhanced proliferation of keratinocytes, selected from the group, consisting of
inflammatory epidermal hyperproliferation conditions, selected from the group, consisting of Lichen ruber and subtypes, Keratosis lichenoides, Lichen simplex chronicus and reactive lichenoid hyperproliferations (e.g. atopic dermatitis), lichenoid reactions at GvHD, ILVEN-Naevus, Lupus erythematodes chron. disc./SCLE/SLE, Pityriasis rubra pilaris, M. Grover, Vitiligo and erythrodermias accompanied by a hyperproliferation of keratinocytes;
non-inflammatory epidermal hyperproliferation conditions;
benign and malign epidermal clonal expansions (e.g. warts, condylomes, actinic keratoses/precanceroses);
benign and malign follicular hyperproliferation conditions, selected from the group, consisting of Keratosis follicularis, Ulerythema ophryogenes, hypertrichoses, trichilemmal cysts, hair follicular cell tumors, proliferating trichilemmal cysts and mixed tumors;
benign and malign epithelial adnex tumors; and
primary and reactive nail cell hyperproliferations.

3. The use according to claim 1 or claim 2, wherein the Xaa-Pro-dipeptide is selected from the group consisting of dipeptide phosphonic acid diaryl esters and dipeptide boronic acids.

4. The use according to claim 3, wherein the dipeptide boronic acid is Pro-Boro-Pro.

5. The use according to claim 1 or claim 2, wherein the Xaa of the amino acid(Xaa)-amide is selected from the group consisting of N^{ε}-4-nitrobenzyloxycarbonyl-L-lysine, L-proline, L-tryptophane, L-isoleucine and L-valine.

6. The use according to claim 1 or claim 2, wherein the cyclic amine is selected from the group consisting of pyrrolidine, piperidine and thiazolidine.

7. The use according to any of claims 1 to 6, wherein the amino acid(Xaa)-amide is selected from the group consisting of
N^{ε}-4-nitrobenzyloxycarbonyl-L-lysine thiazolidide,
N^{ε}-4-nitrobenzyloxycarbonyl-L-lysine pyrrolidide,
N^{ε}-4-nitrobenzyloxycarbonyl-L-lysine piperidide,
N^{ε}-4-nitrobenzyloxycarbonyl-L-lysine 2-cyanothiazolidide,
N^{ε}-4-nitrobenzyloxycarbonyl-L-lysine 2-cyanopyrrolidide and
N^{ε}-4-nitrobenzyloxycarbonyl-L-lysine 2-cyanopiperidide.

8. The use according to any of claims 1 to 7, wherein the DP IV inhibitor is Lys[Z(NO₂)]-thiazolidide, wherein Lys is a L-lysine residue and Z(NO₂) is 4-nitrobenzyloxycarbonyl.

9. The use according to any of claims 1 to 8, wherein the α-aminophosphinic acid is D-Phe-Ψ-[PO(OH)-CH₂]-Phe-Phe.

10. The use according to any of claims 1 and 3 to 9 for the prevention and therapy of asthma bronchiale or hay fever.

11. The use according to any of claims 1 to 10 in combination with per se known carrier substances, auxiliary substances and/or additive substances.

12. The use according to any of claims 1 to 11 for a systemic application for an oral, transdermal, intravenous, subcutaneous, intracutaneous, intramuscular, rectal, vaginal or sublingual administration.

13. The use according to any of claims 1 to 11 for a topical application in the form of creams, ointments, pastes, gels, solutions, sprays, liposomes, agitated mixtures, hydrocolloid dressings, plasters and similar carrier substrates, jet injections and other dermatological bases/vehicles, including instillative applications.

14. The use according to any of claims 1 to 13, wherein the inhibitor of the dipeptidyl peptidase IV (DP IV) or the inhibitor of enzymes having a similar substrate specificity (DP IV-analogous enzymatic activity) and the inhibitor of alanyl-aminopeptidase (aminopeptidase N, APN) or the inhibitor of enzymes having similar substrate specificity (APN-analogous enzymatic activity) in a compartmentally separate formulation are to be administered simultaneously or consecutively in time with the aim of a combined effect.

## Revendications

1. Utilisation d'au moins un inhibiteur, choisi dans le groupe formé par les inhibiteurs de la dipeptidylpeptidase IV (DP IV) et les inhibiteurs d'enzymes ayant la même spécificité de substrat (activité enzymatique analogue à la DP IV),
choisis dans le groupe formé par
- les dipeptides Xaa-Pro ou leurs sels,
Xaa étant un acide α-aminé ou un dérivé à chaîne latérale protégée ;
- les peptides Xaa-Xaa-(Trp)-Pro-(Xaa)ₙ ou leurs sels,
Xaa étant un acide α-aminé et n représentant un nombre entier de 0 à 10;
et
- les amides d'acide aminé (Xaa) ou leurs sels,
Xaa étant un acide α-aminé ou un dérivé à chaîne latérale protégée, et les amines cycliques font fonction de structure amide,
en combinaison avec au moins un inhibiteur, choisi dans le groupe formé par les inhibiteurs de l'alanyl-aminopeptidase (aminopeptidase N, APN) et les inhibiteurs d'enzymes ayant la même spécificité de substrat (activité enzymatique analogue à l'APN),
choisis dans le groupe formé par l'actinonine, la leuhistine, la phebestine, l'amastatine, la bestatine, la probestine, le RB3014, les β-aminothiols, les acides α-aminophosphiniques et leurs sels,
pour préparer un médicament pour la prévention et la thérapie de réactions allergiques du type I.

2. Utilisation d'au moins un inhibiteur, choisi dans le groupe formé par les inhibiteurs de la dipeptidylpeptidase IV (DP IV) et les inhibiteurs d'enzymes ayant la même spécificité de substrat (activité enzymatique analogue à la DP IV),
choisis dans le groupe formé par
- les dipeptides Xaa-Pro ou leurs sels,
Xaa étant un acide α-aminé ou un dérivé à chaîne latérale protégée ;
- les peptides Xaa-Xaa-(Trp)-Pro-(Xaa)ₙ ou leurs sels,
Xaa étant un acide α-aminé et n représentant un nombre entier de 0 à 10;
et
- les amides d'acide aminé (Xaa) ou leurs sels,
Xaa étant un acide α-aminé ou un dérivé à chaîne latérale protégée, et les amines cycliques font fonction de structure amide,
en combinaison avec au moins un inhibiteur, choisi dans le groupe formé par les inhibiteurs de l'alanyl-aminopeptidase (aminopeptidase N, APN) et les inhibiteurs d'enzymes ayant la même spécificité de substrat (activité enzymatique analogue à l'APN),
choisis dans le groupe formé par l'actinonine, la leuhistine, la phebestine, l'amastatine, la bestatine, la probestine, le RB3014, les β-aminothiols, les acides α-aminophosphiniques et leurs sels,
pour préparer un médicament pour la prévention et la thérapie de maladies dermatologiques, qui s'accompagnent d'hyperkératoses folliculaires et épidermiques et d'une prolifération renforcée de kératinocytes, choisies dans le groupe formé par
les états inflammatoires épidermiques d'hyperprolifération, choisis dans le groupe formé par le *lichen ruber* et sous-types, les kératoses lichenoïdes, le lichen simplex chronique, et des hyperproliférations lichenoïdes réactives (par ex. dermatite atopique), les réactions lichenoïdes dans les cas de GvHD, l'ILVEN, le *lupus erythematodes chron. disc.*/SCLE/SLE, la *pityriasis rubra pilaris*, la maladie de Grover, le vitiligo et les érythrodermies accompagnant l'hyperprolifération des kératinocytes ;
les états non-inflammatoires épidermiques d'hyperprolifération ;
les expansions clonales épidermiques circonscrites bénignes et malignes (par exemple, les verrues, les condylomes, les kératoses/précancéroses actiniques) ;
les états folliculaires d'hyperprolifération bénins et malins, choisis dans le groupe formé par la kératose folliculaire, l'ulérythème ophryogène, les hypertrichoses, les kystes trichilemmaux, les tumeurs cellulaires de follicules pileux, les kystes trichilemmaux prolifératifs et les tumeurs mixtes ;
les tumeurs des annexes épithéliales bénignes et malignes ; et
les proliférations de cellules unguéales primaires et réactives.

3. Utilisation selon la revendication 1 ou la revendication 2, dans laquelle le dipeptide Xaa-Pro est choisi dans le groupe formé par les esters diaryliques d'acides dipeptide-phosphoniques et les acides dipeptide-boroniques.

4. Utilisation selon la revendication 3, dans laquelle l'acide dipeptide-boronique est en Pro-Boro-Pro.

5. Utilisation selon la revendication 1 ou la revendication 2, dans laquelle le Xaa de l'amide d'acide aminé (Xaa) est choisi dans le groupe formé par la N^{ε}-4-nitrobenzyloxycarbonyl-L-lysine, la L-proline, le L-tryptophane, la L-isoleucine et la L-valine.

6. Utilisation selon la revendication 1 ou la revendication 2, dans laquelle l'amine cyclique est choisie dans le groupe formé par la pyrrolidine, la pipéridine, et la thiazolidine.

7. Utilisation selon l'une des revendications 1 à 6, dans laquelle l'amide d'acide aminé (Xaa) est choisi dans le groupe formé par le N^{ε}-4-nitrobenzyloxy-carbonyl-L-lysine-thiazolidide, le N^{ε}-4-nitrobenzyloxy-carbonyl-L-lysine-pyrrolidide, le N^{ε}-4-nitrobenzyl-oxy-carbonyl-L-lysine-pipéridide, le N^{ε}-4-nitrobenzyloxy-carbonyl-L-lysine-2-cyanothiazolidide, le N^{ε}-4-nitrobenzyloxy-carbonyl-L-lysine-2-cyanopyrrolidide, le N^{ε}-4-nitrobenzyloxy-carbonyl-L-lysine-2-cyanopipéridide.

8. Utilisation selon l'une des revendications 1 à 7, dans laquelle l'inhibiteur de DP IV est le thiazolidide de Lys[Z(NO₂)], dans lequel Lys représente un radical L-lysine et Z(NO₂) représente le radical 4-nitrobenzyloxy-carbonyle.

9. Utilisation selon l'une des revendications 1 à 8, dans laquelle l'acide α-aminophosphinique est D-Phe-Ψ[PO(OH)-CH₂]-Phe-Phe.

10. Utilisation selon l'une des revendications 1 et 3 à 9, pour la prévention et la thérapie de l'asthme bronchique ou du rhume des foins.

11. Utilisation selon l'une des revendications 1 à 10, en combinaison avec des supports, des adjuvants et/ou des additifs connus en soi.

12. Utilisation selon l'une des revendications 1 à 11, pour l'application systémique à des fins d'application par voie orale, transdermique, intraveineuse, sous-cutanée, intracutanée, intramusculaire, rectale, vaginale ou sublinguale.

13. Utilisation selon l'une des revendications 1 à 11, pour l'application topique sous la forme de crèmes, d'onguents, de pâtes, de gels, de solutions, d'aérosols, de liposomes, de mélanges à agiter, de pansements hydrocolloïdaux, d'emplâtres et de substrats porteurs analogues, d'injections par jets ou d'autres bases/véhicules dermatologiques, y compris une application par instillation.

14. Utilisation selon l'une des revendications 1 à 13, dans laquelle l'inhibiteur de la dipeptidylpeptidase IV (DP IV) ou l'inhibiteur d'enzymes ayant la même spécificité de substrat (activité enzymatique analogue à la DP IV) et l'inhibiteur de l'alanyl-aminopeptidase (aminopeptidase N, APN) ou l'inhibiteur d'enzymes ayant la même spécificité de substrat (activité enzymatique analogue à l'APN), sont administrés dans des formulations séparées dans l'espace, de manière simultanée ou d'une manière telle qu'ils se succèdent directement dans le temps dans le but d'obtenir un effet commun.
